(19) Europäisches Patentamt
European Patent Office
Office européen des brevets

(11) EP 4 745 132 A1

(12) EUROPEAN PATENT APPLICATION
published in accordance with Art. 153(4) EPC

(43) Date of publication:
**20.05.2026 Bulletin 2026/21**

(21) Application number: **24838700.3**

(22) Date of filing: **05.07.2024**

(51) International Patent Classification (IPC):
***C07D 401/14*** (2006.01) ***C07D 401/04*** (2006.01)
***A61K 31/506*** (2006.01) ***A61P 25/00*** (2006.01)
***A61P 25/16*** (2006.01) ***A61P 25/28*** (2006.01)
***A61P 3/00*** (2006.01) ***A61P 3/10*** (2006.01)
***A61P 3/06*** (2006.01) ***A61P 3/04*** (2006.01)
***A61P 35/00*** (2006.01) ***A61P 35/02*** (2006.01)
***A61P 13/12*** (2006.01) ***A61P 11/00*** (2006.01)
***A61P 1/16*** (2006.01) ***A61P 9/00*** (2006.01)
***A61P 17/00*** (2006.01)

(86) International application number:
**PCT/CN2024/103753**

(87) International publication number:
**WO 2025/011447 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.07.2023 CN 202310860161**

(71) Applicant: **Technoderma Medicines Inc.**
**Chengdu, Sichuan 610219 (CN)**

(72) Inventors:
- **FANG, Wenkui Ken**
  **California 92618 (US)**
- **LI, Guanqun**
  **Oregon 97239 (US)**
- **CAI, Yuting**
  **Chengdu, Sichuan 610219 (CN)**
- **WANG, Zengquan**
  **Nevada 89113 (US)**

(74) Representative: **J A Kemp LLP**
**80 Turnmill Street**
**London EC1M 5QU (GB)**

# (54) PYRIMIDINAMINE NUAK INHIBITOR, AND PREPARATION METHOD THEREFOR AND USE THEREOF

(57) Provided in the present application is a pyrimidinamine compound, which is characterized in that the pyrimidinamine compound is a compound as shown in formula I, or a stereoisomer, tautomer, isotopic derivative, hydrate, solvate, prodrug and pharmaceutically acceptable salt thereof. The pyrimidinamine compound of the present application has an excellent NUAKI/NUAK2 kinase inhibitory activity, and can be used for preventing or treating the following diseases by inhibiting NUAK1/NUAK2: neuropsychiatric diseases, metabolic diseases, tumors, visceral fibrotic diseases and skin fibrotic diseases.

EP 4 745 132 A1

## Description

### CROSS-REFERENCE TO RELATED APPLICATIONS

The present application claims priority to Chinese Patent Application No. 202310860161.7, filed on July 13, 2023, which is incorporated herein by reference in its entirety..

### TECHNICAL FIELD

The present disclosure relates to the field of small molecule compounds, specifically to a pyrimidinamine-based NUAK inhibitor and a preparation method and use thereof. The compound, by inhibiting NUAK1/NUAK2, is useful for the prevention or treatment of the following diseases: neuropsychiatric diseases, metabolic diseases, tumors, visceral fibrotic diseases, and skin fibrotic diseases, or used solely for alleviating traumatic and postoperative scars.

### BACKGROUND

Protein kinases are a group of important functional proteins participating in the regulation of metabolism, polarity, growth, division, and differentiation of cells, and the human genome encodes more than 500 types of protein kinases that can transfer phosphate groups from ATP (adenosine triphosphate) to specific serine, threonine, or tyrosine residues of substrate proteins for phosphorylation. Most protein kinases are serine/threonine kinases, and there are less than 100 types of tyrosine kinases. Adenosine monophosphate-activated protein kinase (AMPK) belongs to serine/threonine kinases (STK) and is an important regulator of cellular energy homeostasis in mammals. AMPK can regulate glucose and lipid metabolism, cell proliferation, and cell polarity by sensing a change in an intracellular AMP (Adenosine monophosphate)/ATP or ADP (adenosine diphosphate)/ATP ratio under metabolic stress (for example, hypoxia or heat shock), and therefore, can be referred to as a metabolic sensor protein. AMPK is evolutionarily highly conserved and a heterotrimeric protein consisting of an $\alpha$ subunit (containing a kinase domain, KD), a $\beta$ subunit (regulating kinase activity), and a $\gamma$ subunit. The $\gamma$ subunit contains four cystathionine-$\beta$-synthase (CBS) domains and is responsible for detecting changes in the intracellular AMP/ATP and ADP/ATP ratios (Hardie DG, Trends in Cell Biology. 2016, 26:190).

AMPK dysfunction can cause a variety of diseases such as obesity, diabetes, inflammatory diseases and tumors, and therefore, regulation of AMPK function by the body is critical. First of all, phosphorylation of threonine 172 (T172) on the $\alpha$ subunit by upstream kinases is required for AMPK activation. Three types of upstream kinases of AMPK have been found by now, namely, liver kinase B1 (LKB1), $Ca^{2+}$/calmodulin-dependent PK kinase 2 (CaMKK2) and transforming growth factor-$\beta$-activated kinase 1 (TAK1). Corresponding to the activation of AMPK via phosphorylation of the T172 site by the upstream kinase, three protein phosphatases are found to inhibit the activity of AMPK by removing a phosphate group from T172, including protein phosphatase 2A (PP2A), protein phosphatase 2C (PP2C) and $Mg^{2+}$/$Mn^{2+}$-dependent protein phosphatase 1E (PPM1E). When cells are in a low-energy state (high AMP/ATP ratio or ADP/ATP ratio), the $\alpha$ subunit KD and $\gamma$ subunit of AMPK are tightly cross-linked, while the $\beta$ subunit is myristoylated, preventing protein phosphatases from accessing the T172 site and thus maintaining AMPK activation. In addition, when cells are in a high-energy state, KD and $\gamma$ subunit are detached, and T172 is exposed to phosphatase, thereby causing inactivation of AMPK (Steinberg GR, Nature Reviews Drug Discovery. 2019, 18:527).

In addition to AMPK upstream kinases and protein phosphatases that can activate and inactivate AMPK respectively, there are also a type of AMPK-related kinases (ARK) participating in the regulation of AMPK functions, and a total of 12 ARKs (BRSK1, BRSK2, NUAK1, NUAK2, QIK, QSK, SIK, MARK1, MARK2, MARK3, MARK4 and MELK) have been found by now and all belong to serine/threonine kinases. Kinase domains of ARKs have high homology with the $\alpha$ subunit of AMPK. All ARKs can be activated by LKB1 except MELK, their kinase-activating phosphorylation sites are equivalent to AMPK T172, and ARKs also functionally participate in the regulation of metabolism, proliferation and polarity of cells. However, unlike AMPK with regulatory subunits, ARKs cannot be directly regulated by the intracellular AMP/ATP ratio (Bright NJ, Acta Physiologica. 2009, 196:15).

ARKs are classified into several ARK subfamilies by different protein structures and functional characteristics. NUAK subfamily (Nu (novel) and AMPK-related kinase) of ARKs includes two members of NUAK1 (initially named ARK5) and NUAK2 (initially named sucrose nonfermenting-like/AMPK related kinase, SNARK). Amino acid sequences of the two members are approximately 55% homologous, and molecular weights of NUAK1 and NUAK2 estimated according to amino acid sequences are 76 kDa and 69 kDa respectively. They share highly similar protein structures, with an N-terminal kinase domain and a C-terminal ubiquitin-binding domain, and currently, it is yet unclear whether NUAK is a heterotrimeric structure like AMPKs. NUAK1 and NUAK2 are expressed in most tissues, an expression level of NUAK1 in organs and tissues such as brain, skin, muscle, upper gastrointestinal tract and endocrine is significantly higher than that in other parts, organs and tissues with the highest expression level of NUAK2 are the gastrointestinal tract, female reproductive system, skin, bone, brain, endocrine and the like, and the expression of NUAK2 shows more tissue specificity. It should be noted

that NUAK1 and other ARKs and AMPK proteins are mainly distributed in cytoplasm, while NUAK2 is mainly distributed in nucleus, and there are indications that NUAK2 participates in the expression of genes related to metabolic stress as a transcriptional regulator (Sun X, J of Molecular Endocrinology. 2013, 51:R15).

As serine/threonine kinases, NUAK1 and NUAK2 can phosphorylate a variety of protein substrates, including proteins participating in cell signaling and metabolism, cell proliferation, cell apoptosis and autophagy, and cytoskeletal tissues. As it is known, NUAK1 can phosphorylate AMPK, LATS1/2, p53 tumor suppressor protein, and myosin phosphatase target subunit 1 (Mypt1) regulating actin cytoskeletal tissue. NUAK2 can phosphorylate transcription factors Gli3 and FoxO1 of Hedgehog signaling pathway, kinesin light chain 1 (KLC1), Rho GDP dissociation inhibitor $\alpha$ (Rho GDI$\alpha$), and the like.

The functional regulation of NUAK1 and NUAK2 relates to a variety of intracellular signaling systems. In addition to being activated via phosphorylation by LKB1 (T211, equivalent to T172 of AMPK) and activated by $Ca^{2+}$/PKC, NUAK1 is the only member of the AMPK-related protein family that can be activated by Akt, and an increase in the activity of NUAK1 is also found in the activation of signaling pathways of some growth factors such as insulin-like growth factor 1 (IGF1). Contraction states of skeletal muscle cells are also accompanied by increased activity of NUAK1. Both NUAK1 and NUAK2 can be activated by LKB1 (T211/NUAK1, T208/NUAK2, equivalent to T172 of AMPK), but NUAK2 can be autophosphorylated, and NUAK2 can interact with ubiquitin-specific protease 9, X-linked (USP9X), which is a deubiquitinase and can maintain activity of NUAK2. Different intracellular stimuli such as low osmotic pressure, DNA damage, oxidation and nutrient deficiencies can all lead to the activation of NUAK2, and the activation of NUAK2 is also found in the contraction of skeletal muscle cells (Brooks D, Data in Brief. 2022, 43:108482).

A number of studies have revealed that NUAK plays an important role in pathogenesis of diseases such as metabolic diseases, tumors, neurodegenerative diseases and fibrotic diseases, homozygous NUAK1/NUAK2 knockout mice basically die in the embryonic period, mice with heterozygous NUAK1 gene deleted exhibit systemic and nervous tissue hypoplasia (for example, cerebral cortex and peripheral neurons), heterozygous gene mutation of human NUAK1 is associated with autism spectrum disorders (ASD), cognitive deficits, attention deficit/hyperactivity disorder (AD/HD) and schizophrenia. Deletion of the human NUAK2 gene causes anencephaly, which is a severe neural tube malformation that causes defective fetal development and whose pathogenesis is related to loss of YAP function (Bonnard C, J Exp Med. 2020, 217:e20191561). Skeletal muscle cell-specific knockout of the NUAK1 gene can avoid high-fat diet-induced subclinical diabetes. However, a phenotype of mice with heterozygous NUAK2 gene deleted is similar to a series of clinical manifestations of human type II diabetes mellitus with obesity, and these phenomena may be related to imbalance of cellular autophagy mechanism (Bennison SA, Cellular Signaling. 2022, 100:110472; Blazejewski SM, Scientific Reports. 2011, 11:8156).

Activation of NUAK1 caused by Akt and other protein kinases can promote survival of tumor cells in an environment with energy deficiency and protect tumor cells from apoptosis, and NUAK2 also inhibits, through a similar mechanism, apoptosis induced by TNF $\alpha$ and CD95. In addition, after activation, NUAK1 promotes invasion and metastasis of tumor cells by upregulating the matrix metalloproteinases (MMP), while NUAK2 participates in occurrence of tumor metastasis by enhancing tumor cell motility (Hou X, Oncogen. 201, 30:2933; Chen Y, Cell Death and Disease. 2020, 11:712; Molina E, Cells. 10:2760; Humbert N, The EMBO Journal. 2010, 29:376). A lot of evidence reveals that NUAK1 and NUAK2 play a role in tumor formation and metastasis, NUAK2 has a stronger function in promoting tumor formation and metastasis than NUAK1, and NUAK2 inhibitors should be more likely to become new targeted antitumor drugs than NUAK1 inhibitors.

Recent studies have revealed that NUAK plays a critical role in development of tissue fibrosis through interaction with transforming growth factor-$\beta$ (TGF-$\beta$) signaling pathway. First, TGF-$\beta$ can upregulate gene transcription of NUAK1 and NUAK2 in a variety of epithelial cells such as keratinocytes and human dermal fibroblasts, while inactivation of MAPK (ERK1/2 and p38) signals inhibits expression of TGF-$\beta$-dependent NUAK2. In addition, NUAK2 inhibits degradation of SMAD3 in cells by binding to the structurally crosslinking domain and MH2 domain of the intracellular signaling mediator SMAD3 protein of TGF-$\beta$. A similar mechanism exists between NUAK2 and T$\beta$RI. TGF-$\beta$-induced expression of genes encoding profibrotic molecules such as fibronectin (FN), plasminogen activator inhibitor 1 (PAI1) and tissue inhibitor of metalloproteinase-1 (TIMP1) depends on the presence of NUAK2. These findings indicate that NUAK2 promotes fibrogenesis. Studies have also revealed that NUAK1 promotes fibrosis of kidney, lung and liver by upregulating two signaling pathways of TGF-$\beta$ and YAP, and in animal experiments, inhibition of NUAK1 can alleviate scars and mature scar tissues caused by new trauma (Gill MK, Nature Communications. 2018. 9:3510). Interestingly, studies have revealed that when expression of genes encoding FN in keratinocytes with NUAK1 gene knocked out is upregulated, NUAK1 may inhibit fibrosis by influencing TGF-$\beta$ signaling through a negative feedback mechanism (van de Vis RAJ, Cancers. 2021, 13:3377). In general, inhibition of NUAK may effectively inhibit fibrosis processes of tissues and organs involved.

Given their structural and functional properties, kinases have long been ideal targets for small-molecule drugs, and NUAK1 and NUAK2 are involved in the occurrence of a variety of diseases, some NUAK inhibitors have been in an early stage of development by now, but their efficacy and safety are yet undetermined (Banerjee S, The Biochemical Journal. 2014, 457:215). Therefore, development of selective NUAK inhibitors and dual-target NUAK1/2 inhibitors is

expected to provide a new direction for innovative treatments of neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars. Developing next-generation NUAK inhibitors has significant potential clinical application value.

## SUMMARY OF THE INVENTION

An objective of the present disclosure is to obtain an effective NUAK1/NUAK2 inhibitor that is useful for preparing medicaments for prevention or treatment of the following diseases: neuropsychiatric diseases (for example, Parkinson's disease and Alzheimer's disease), metabolic diseases (for example, diabetes, hyperlipidemia and obesity), tumors (for example, liver cancer, leukemia, lymphoma and tumor metastasis), visceral fibrotic diseases (for example, liver cirrhosis, renal fibrosis, pulmonary fibrosis and post-myocarditis sequelae), and skin fibrotic diseases (for example, scleroderma, keloids and hypertrophic scars), or solely for alleviating traumatic and postoperative scars.

To achieve the foregoing objective, an aspect of the present disclosure provides a pyrimidinamine-based compound, where the pyrimidinamine-based compound is a compound represented by Formula I shown below, or a stereoisomer, a geometric isomer, a tautomer, an isotopic derivative, a hydrate, a solvate, a prodrug, or a pharmaceutically acceptable salt thereof;

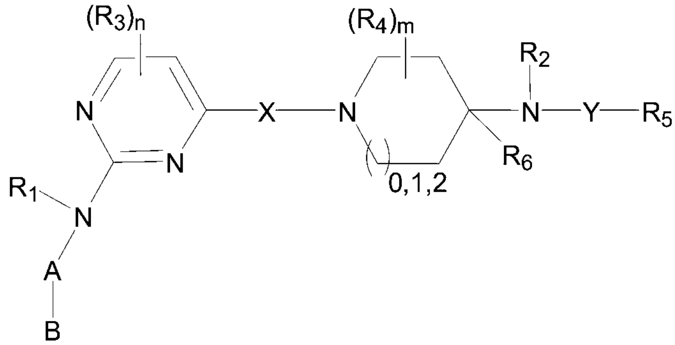

Formula I

where X is a direct bond, -O-, -S-, -$NR_7$- or -$CR_8R_9$-, and Y is -CO- or -$S(O)_2$-;

A is an optionally substituted 6- to 10-membered aryl group or 5- to 10-membered heteroaryl group, B is an optionally substituted 3- to 10-membered cycloalkyl group or 3- to 10-membered heterocycloalkyl group, and the substituent on A or B is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;

$R_1$, $R_2$, $R_6$, $R_7$, $R_8$, and $R_9$ are each independently H or an optionally substituted C1-C8 alkyl group;

$R_3$ and $R_4$ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;

$R_5$ is an optionally substituted 3- to 10-membered cycloalkyl group or an optionally substituted 3- to 10-membered heterocycloalkyl group, where the substituent on the cycloalkyl group or heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;

the substituent on the C1-C8 alkyl group is one or more selected from the group consisting of halogen, an amino group, a hydroxyl group, a nitro group, a cyano group, and a sulfhydryl group; and

n is 0, 1, or 2, and m is 0, 1, 2, 3, 4, or 5.

In one set of embodiments, $R_5$ is an optionally substituted 3- to 10-membered cycloalkyl group, preferably an optionally substituted 3- to 6-membered cycloalkyl group, more preferably a 3- to 6-membered cycloalkyl group substituted with halogen, and even more preferably, $R_5$ is a 3- to 6-membered cycloalkyl group substituted with fluorine.

Further, $R_5$ is

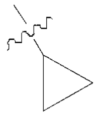

,

or

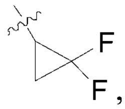

,

preferably $R_5$ is

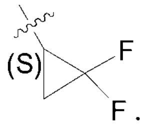

.

In one set of embodiments, $R_6$ is a methyl group.

In one set of embodiments, $R_2$ is H.

In one set of embodiments, B is an optionally substituted 3- to 6-membered heterocycloalkyl group, preferably an optionally substituted 5- to 6-membered heterocycloalkyl group, more preferably, B is an optionally substituted piperidinyl group or an optionally substituted piperazinyl group.

In one set of embodiments, the compound represented by Formula I is represented by Formula I-1 or Formula I-2:

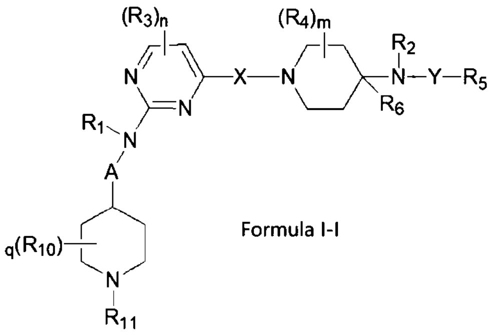

Formula I-1

,

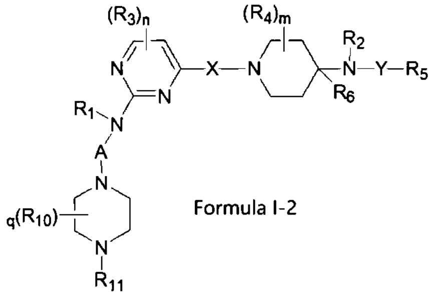

Formula I-2

$R_{10}$ is selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;

$R_{11}$ is H or an optionally substituted C1-C8 alkyl group;

the substituent on the C1-C8 alkyl group is one or more selected from the group consisting of halogen, an amino group, a hydroxyl group, a nitro group, a cyano group, and a sulfhydryl group; and

q is 0, 1, 2, 3, or 4.

Further, $R_{11}$ is H or a methyl group.

In one set of embodiments, A is an optionally substituted phenyl group or an optionally substituted 5-membered heteroaryl group.

Further, A is one of the following optionally substituted moieties:

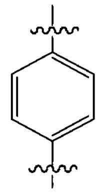
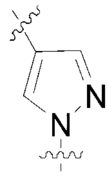
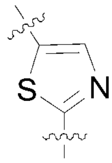

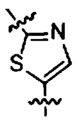
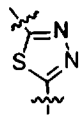
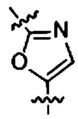
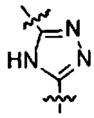
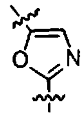
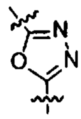
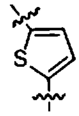

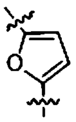
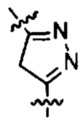
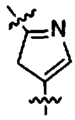
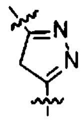
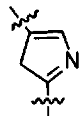
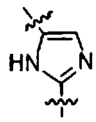
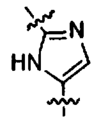

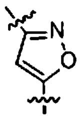
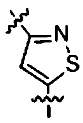
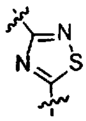
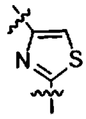
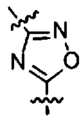
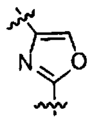 ,

preferably, the upper position is attached to the amino group, and the lower position is attached to the moiety B.

In one set of embodiments, $R_3$ is chloro, preferably at the para-position to the amino group.

In one set of embodiments, the substituent on ring A is a methoxy group, preferably at the ortho-position to the amino group.

In one set of embodiments, the compound represented by Formula I is one of the following compounds:

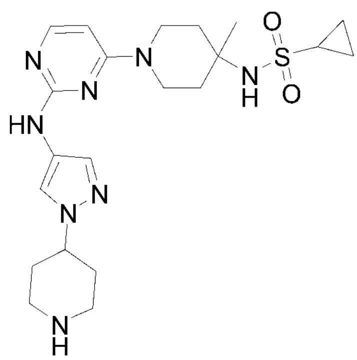
TDM-181137

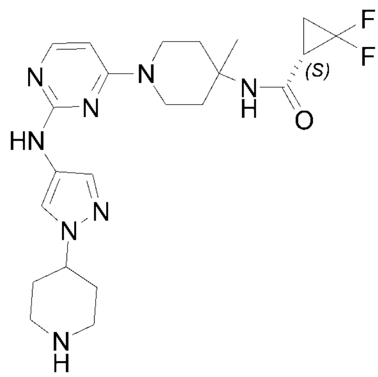
TDM-181163

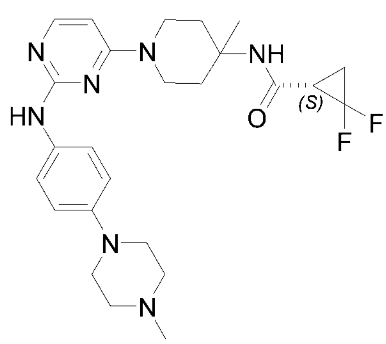
TDM-181164

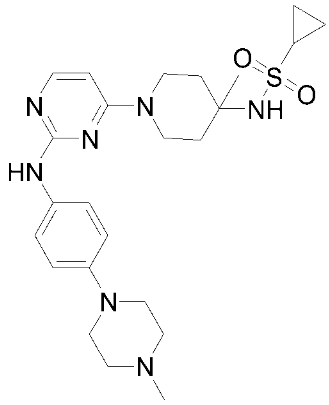

TDM-181165

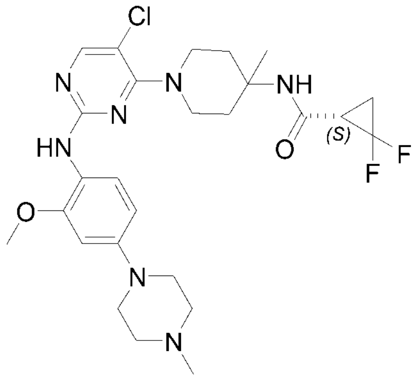

TDM-181172

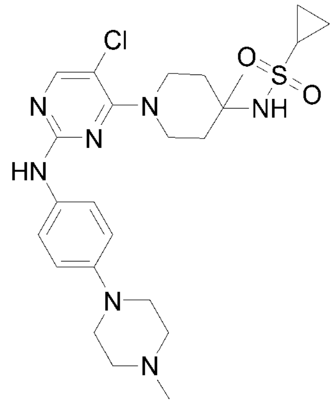

TDM-181173

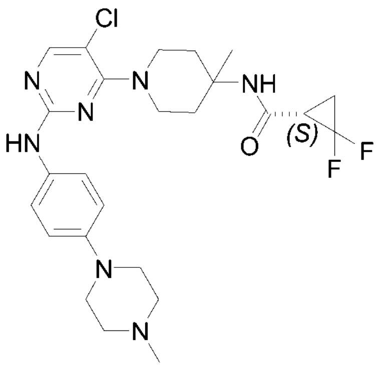

TDM-181174

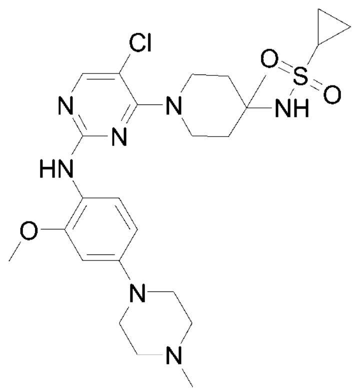

TDM-181175

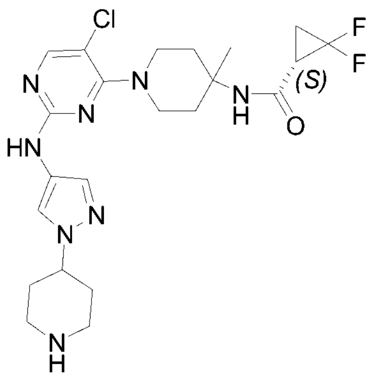

TDM-181178

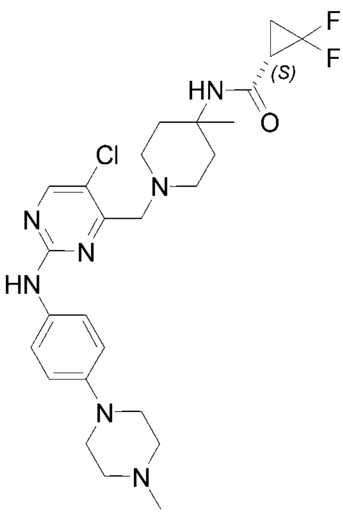

TDM-181182

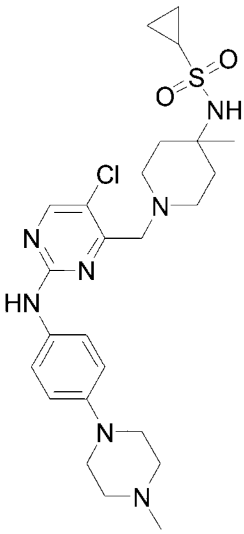

TDM-181183

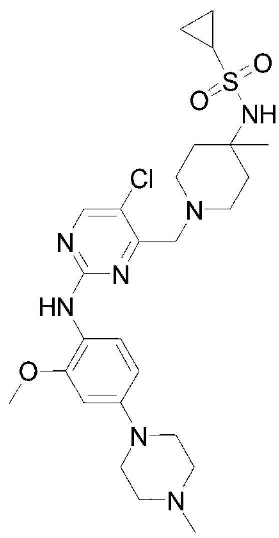

TDM-181184

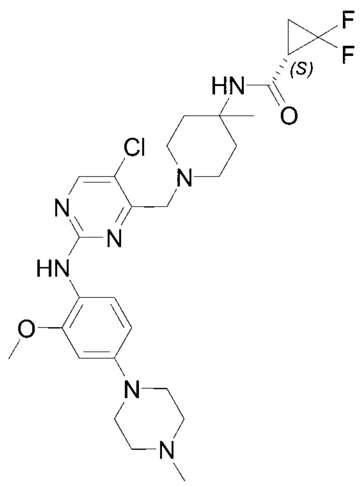

TDM-181185

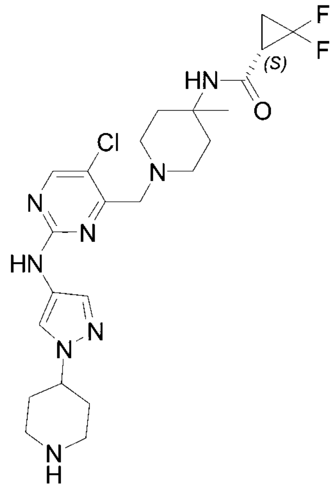

TDM-181186 .

In one set of embodiments, the pharmaceutically acceptable salt is a formate salt.

In one set of embodiments, the isotopic derivative is a deuterated compound.

Another aspect of the present application further provides a method for preparing the pyrimidinamine-based compound, including: preparing the compound of Formula I from a compound of Formula II and a compound of Formula III; or

preparing a compound of Formula V from a compound of Formula IV and a compound of Formula II, and then preparing the compound of Formula I from the compound of Formula V;
optionally, the preparation process includes necessary protection and deprotection steps;

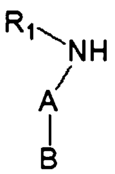

Formula II

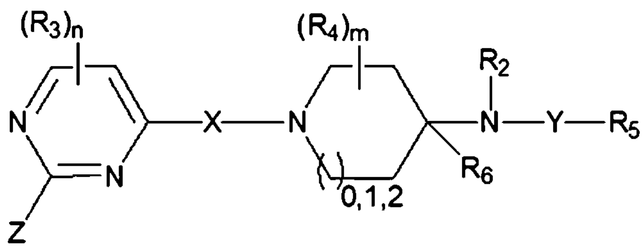

Formula III

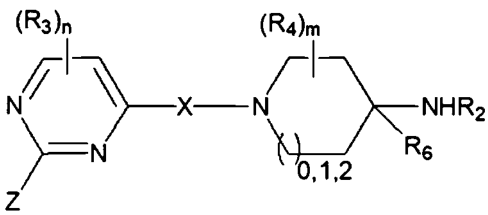

Formula IV

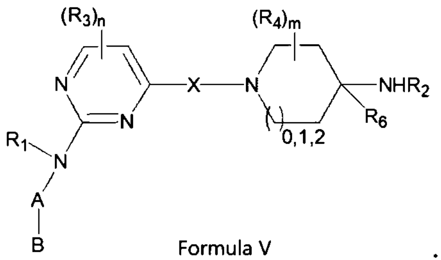

Formula V ;

where Z is a leaving group, preferably halogen, more preferably Cl, and other groups are as defined above.

In one set of embodiments, the amino or imino group on Ring B in Formula II is first protected, the protected compound is then coupled with a compound of Formula III, followed by removing the protecting group to obtain the compound of Formula I.

In one set of embodiments, the amino or imino group attached to $R_2$ in the compound of Formula IV is first protected, and the amino group or the imino group on the ring B in Formula II is optionally protected. The protected compound of Formula IV is then coupled with the optionally protected compound of Formula II, followed by removing the protecting group on the amino or imino group attached to $R_2$ to obtain the compound of Formula V. The compound of Formula V is subsequently reacted with $R_5$-Y-OH, and the protecting group on ring B is optionally removed to obtain the

compound of Formula I.

The present application provides a pharmaceutical composition, in which the pyrimidinamine-based compound according to the present application is used as an active ingredient, and the pharmaceutical composition further contains a pharmaceutically acceptable carrier or excipient.

The present application provides the use of the pyrimidinamine-based compound in the preparation of a NUAK1 or NUAK2 inhibitor.

The present application provides the use of the pyrimidinamine-based compound in preparation of a medicament, wherein the compound is useful for the prevention or treatment of the following diseases by inhibiting NUAK1 or NUAK2: neuropsychiatric diseases, metabolic diseases, tumors, visceral fibrotic diseases, or skin fibrotic diseases.

In one set of embodiments, the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar; or the compound is used solely for alleviating a traumatic or postoperative scar.

Beneficial effects of the present disclosure:

The present disclosure provides a class of pyrimidinamine-based compounds, and *in vitro* kinase activity inhibition assays show that the compounds according to the present disclosure have excellent inhibitory activity against NUAK1/NUAK2 kinases. The compounds are useful for the prevention or treatment of the following diseases by inhibiting NUAK1 or NUAK2: neuropsychiatric diseases, metabolic diseases, tumors, visceral fibrotic diseases, or skin fibrotic diseases.

## DETAILED DESCRIPTION

The present disclosure is described in detail hereinafter. It should be understood that the specific embodiments described herein are intended merely to illustrate and explain the present disclosure, and are not intended to limit the present disclosure.

The endpoints and any values of the ranges disclosed herein are not limited to those precise ranges or values, and such ranges or values should be understood to include values approximating those ranges or values. For numerical ranges, combinations may be made between the endpoint values of each range, between the endpoint values of each range and individual point values, and between individual point values to obtain one or more new numerical ranges, and such numerical ranges are to be regarded as specifically disclosed herein.

Before describing the present disclosure in detail, it should be understood that the terms used herein are for the purpose of describing particular embodiments only and are not intended to limit the scope of the present disclosure, which is to be limited solely by the appended claims. For a more complete understanding of the present disclosure described herein, the following terms are employed, with definitions set forth below. Unless otherwise defined, all technical and scientific terms used herein have the same meaning as commonly understood by one of ordinary skill in the art to which this disclosure belongs.

Definitions

Unless otherwise specified, the following terms as used in the present disclosure have the definitions set forth below.

Features illustrated or described as part of one embodiment or group of embodiments may be used in another embodiment or group of embodiments to produce further embodiments.

In the present disclosure,

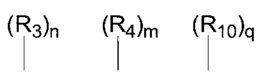

on a cyclic moiety indicates that n $R_3$, m $R_4$, or q $R_{10}$ may be attached at any possible position on the cyclic moiety.

In the present disclosure,

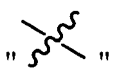

in some substituents represents the point of attachment.

Unless otherwise specified, the term "optionally substituted" means that the hydrogen atoms on the substituted group are unsubstituted, or one or more substitutable positions on the substituted group are each independently substituted with substituent(s) each independently selected from the group consisting of deuterium, halogen, an amino group, a hydroxyl group, a nitro group, a cyano group, a sulfhydryl group, oxo, an optionally substituted C1-C8 alkyl group,

an optionally substituted C1-C8 alkoxy group, an optionally substituted C1-C8 alkylthio group, and an optionally substituted C1-C8 alkylamino group. The substituent on the C1-C8 alkyl group is one or more selected from the group consisting of deuterium, halogen, an amino group, a hydroxyl group, a nitro group, a cyano group, and a sulfhydryl group. When the substituent is "oxo", it means that two hydrogen atoms at the same substitution position are replaced by an oxygen atom.

The term "aryl group" refers to a monocyclic or bicyclic aromatic carbocyclic system containing from 6 to 10 carbon atoms. Examples of aryl groups include a phenyl group and a naphthyl group.

The term "heteroaryl group" refers to an aromatic monocyclic or polycyclic ring system having from 5 to 10 ring atoms, preferably from 5 to 8 ring atoms, more preferably from 5 to 6 ring atoms, where one, two, three, four or more ring atoms are heteroatoms and the remaining ring atoms are carbon atoms, where the heteroatoms are each independently selected from O, N, or S, and the number of heteroatoms is preferably 1, 2, 3, or 4. The heteroaryl may be a 5- to 6-membered aromatic monocyclic ring containing 1 or 2 heteroatoms selected from N or S, or a 5- to 6-membered aromatic monocyclic ring containing 1 or 2 N atoms. Examples of heteroaryl groups include, but are not limited to, a furanyl group, a thienyl group, an oxazolyl group, a thiazolyl group, an isoxazolyl group, an oxadiazolyl group, a thiadiazolyl group, a pyrrolyl group, a pyrazolyl group, an imidazolyl group, a triazolyl group, a tetrazolyl group, a pyridinyl group, a pyrimidinyl group, a pyrazinyl group, a pyridazinyl group, a thiodiazolyl group, a triazinyl group, a phthalazinyl group, a quinolinyl group, an isoquinolinyl group, a pteridinyl group, a purinyl group, an indolyl group, an isoindolyl group, an indazolyl group, a benzofuranyl group, a benzothienyl group, a benzopyridinyl group, a benzopyrimidinyl group, a benzopyrazinyl group, a benzimidazolyl group, a benzophthalazinyl group, a pyrrolo[2,3-b]pyridinyl group, an imidazo[1,2-a]pyridinyl group, a pyrazolo[1,5-a]pyridinyl group, a pyrazolo[1,5-a]pyrimidinyl group, an imidazo[1,2-b]pyridazinyl group, a [1,2,4]triazolo [4,3-b]pyridazinyl group, a [1,2,4]triazolo[1,5-a]pyrimidinyl group and a [1,2,4]triazolo[1,5-a]pyridinyl group.

The term "cycloalkyl group" refers to a saturated monocyclic, bicyclic, or tricyclic hydrocarbon system containing from 3 to 10 carbon atoms, wherein the monocyclic, bicyclic, or tricyclic system contains no aromatic ring. Bicyclic groups include bridged rings, spiro rings, fused rings, and the like. Preferably, the cycloalkyl group contains from 3 to 10 carbon atoms (C3-10 cycloalkyl), more preferably from 3 to 8 carbon atoms (C3-8 cycloalkyl), from 3 to 6 carbon atoms (C3-6 cycloalkyl), from 4 to 6 carbon atoms (C4-6 cycloalkyl), or from 5 to 6 carbon atoms (C5-6 cycloalkyl). Examples include, but are not limited to, a cyclopropyl group, a cyclobutyl group, a cyclopentyl group, a cyclohexyl group, a methylcyclopropyl group, a 2-ethyl-cyclopentyl group, and a dimethylcyclobutyl group.

The term "heterocycloalkyl group" refers to a saturated monocyclic, bicyclic, or polycyclic hydrocarbon group, preferably having from 3 to 10 ring atoms, where one, two, three or more ring atoms are selected from N, O, and S, and the remaining ring atoms are carbon atoms, including bridged rings, spiro rings, fused rings, and the like. Preferably, the heterocycloalkyl group has from 3 to 8 ring atoms (3- to 8-membered heterocycloalkyl), from 3 to 6 ring atoms (3- to 6-membered heterocycloalkyl), from 4 to 6 ring atoms (4- to 6-membered heterocycloalkyl), or from 5 to 6 ring atoms (5- to 6-membered heterocycloalkyl). The number of heteroatoms is preferably from 1 to 4, more preferably from 1 to 3 (i.e., 1, 2, or 3). The heterocycloalkyl may be a 5- to 6-membered monocyclic heterocycloalkyl containing 1 or 2 N atoms. Examples of heterocycloalkyl groups include a pyrrolidinyl group, an imidazolidinyl group, a tetrahydrofuranyl group, a piperidinyl group, a piperazinyl group, a pyranyl group, an aziridinyl group, an oxiranyl group, a thiiranyl group, an azetidinyl group, an oxetanyl group, a thietanyl group, oxacyclohexyl, a morpholinyl group, a thiomorpholinyl group, a dioxanyl group, a dithianyl group, an oxazolidinyl group, a thiazolidinyl group, a pyrazolidinyl group, an imidazolinidinyl group, and the like.

The term "alkyl group" refers to a monovalent saturated aliphatic hydrocarbon group, preferably a straight or branched chain group containing from 1 to 8 carbon atoms (C1-8 alkyl; the number of carbon atoms is 1, 2, 3, 4, 5, 6, 7, or 8), more preferably from 1 to 6 carbon atoms (C1-6 alkyl; the number of carbon atoms is 1, 2, 3, 4, 5, or 6). Examples include, but are not limited to, a methyl group, an ethyl group, an n-propyl group, an isopropyl group, an n-butyl group, an isobutyl group, a tert-butyl group, a sec-butyl group, an n-pentyl group, a neopentyl group, a 1,1-dimethylpropyl group, a 1,2-dimethylpropyl group, a 2,2-dimethylpropyl group, a 1-ethylpropyl group, a 2-methylbutyl group, a 3-methylbutyl group, an n-hexyl group, an n-heptyl group, and an n-octyl group.

The terms "alkoxy group", "alkylthio group", and "alkylamino group" refer to -O-alkyl group, -S-alkyl group, -NH-alkyl group or dialkylamino group, respectively, where the alkyl group is as defined above. Representative examples include, but are not limited to, a methoxy group, an ethoxy group, a propoxy group, an isopropoxy group, a butoxy group, a 1-methylpropoxy group, a 2-methylpropoxy group, a tert-butoxy group, a methylthio group, an ethylthio group, a propylthio group, an isopropylthio group, a butylthio group, a 1-methylpropylthio group, a 2-methylpropylthio group, a tert-butylthio group, a methylamino group, an ethylamino group, a propylamino group, a dimethylamino group, a diethylamino group, a dipropylamino group, and a methylethylamino group.

The term "halogen" refers to F, Cl, Br, and I.

The active compound of the present disclosure is understood to include the compound itself, as well as its stereoisomers, tautomers, isotopic derivatives, hydrates, solvates, prodrugs, or pharmaceutically acceptable salts thereof. The stereoisomers, tautomers, hydrates, solvates, prodrugs, and pharmaceutically acceptable salts of the compound may be prepared by conventional techniques in the art, and exert the same or similar effects *in vitro* and *in vivo*

through substantially the same mechanism of action as said compound.

The term "stereoisomer" refers to isomers arising from the different spatial arrangement of atoms in a molecule, including configurational isomers and conformational isomers. Configurational isomers further include geometric isomers (or cis-trans isomers) and optical isomers (including enantiomers and diastereomers). Geometric isomers may exist in the present compounds. Optical isomers are substances having identical molecular structures, similar physicochemical properties, but different optical activities. The compounds according to the present disclosure may contain asymmetrically substituted carbon atoms in either the R or S configuration, wherein the terms "R" and "S" are as defined in IUPAC 1974 Recommendations for Section E, Fundamental Stereochemistry, Pure Appl. Chem. (1976) 45, 13-10. Compounds containing asymmetrically substituted carbon atoms (with equal amounts of R and S configurations) are racemic at those carbon atoms. An excess of one configuration relative to the other results in a higher proportion of that configuration, preferably an excess of about 85% to 90%, more preferably an excess of about 95% to 99%, even more preferably an excess of greater than about 99%. Accordingly, the present disclosure includes racemic mixtures, individual relative and absolute optical isomers, and mixtures of relative and absolute optical isomers.

The term "tautomer" refers to structural isomers having different energies that are interconvertible via a low energy barrier. Where tautomerization is possible (e.g., in solution), a chemical equilibrium of tautomers can be reached. For example, proton tautomers (also called prototropic tautomers) include interconversions via migration of a proton, such as keto-enol tautomerization and imine-enamine tautomerization. Valence tautomers include interconversions via reorganization of some bonding electrons.

The term "isotopic derivative" means that the compound of the present disclosure may exist in an isotopically labeled or enriched form, containing one or more atoms whose atomic weight or mass number differs from the atomic weight or mass number of the most abundant naturally occurring atom. Isotopes may be radioactive or non-radioactive. Isotopes of hydrogen, carbon, phosphorus, sulfur, fluorine, chlorine, and iodine include, but are not limited to, $^{2}H$, $^{3}H$, $^{13}C$, $^{14}C$, $^{15}N$, $^{18}O$, $^{32}P$, $^{35}S$, $^{18}F$, $^{36}Cl$, and $^{125}I$. Compounds containing other isotopes of these and/or other atoms are within the scope of the present disclosure. The isotopically labeled compounds of the present disclosure may be prepared by general methods known to those of ordinary skill in the art.

The term "hydrate" refers to an association formed by one or more water molecules and the compound of the present disclosure.

The term "solvate" refers to an association formed by one or more solvent molecules and the compound of the present disclosure.

The term "prodrug" refers to a derivative of an active drug designed to improve certain identified undesirable physical or biological properties. Physical properties typically relate to solubility (excessively high or insufficient lipid or aqueous solubility) or stability, while problematic biological properties include overly rapid metabolism or poor bioavailability, which may themselves be related to physicochemical properties.

The term "pharmaceutically acceptable salt" refers to a salt that is, within the scope of sound medical judgment, suitable for use in contact with the tissues of mammals, especially humans, without undue toxicity, irritation, allergic response, and the like, and commensurate with a reasonable benefit/risk ratio. When the compound is basic, pharmaceutically acceptable salts include salts formed from inorganic acids and salts formed from organic acids. When the compound is acidic, pharmaceutically acceptable salts include salts formed from inorganic bases and/or organic bases.

The term "pharmaceutically acceptable carrier" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, emulsifier, or the like that is approved by a relevant governmental regulatory authority for use in humans or livestock.

As used herein, the term "treatment" refers to any administration of a therapeutic agent according to a therapeutic regimen that achieves a desired effect, i.e., partial or complete reduction, amelioration, alleviation, inhibition, delay of onset, reduction in severity, and/or reduction in the incidence of one or more symptoms or features of a particular disease, disorder, and/or condition. In some embodiments, administration of a therapeutic agent according to a therapeutic regimen is associated with the achievement of the desired effect. Such treatment may be administered to a subject who does not exhibit signs of the relevant disease, disorder, and/or condition and/or to a subject who exhibits only early signs of the disease, disorder, and/or condition. Alternatively or additionally, such treatment may be administered to a subject who exhibits one or more established signs of the relevant disease, disorder, and/or condition. In some embodiments, treatment may be administered to a subject diagnosed with the relevant disease, disorder, and/or condition. In some embodiments, treatment may be administered to a subject known to have one or more predisposing factors that are statistically associated with an increased risk of developing the relevant disease, disorder, and/or condition.

According to the present disclosure, the medicament prepared for the pharmaceutical use described herein may contain, in addition to the pyrimidinamine-based compound of the present disclosure as an active ingredient, one or more other agents useful for the prevention or treatment of the relevant diseases as a further active ingredient. When the medicament contains multiple active ingredients, the active ingredients may be administered simultaneously, sequentially, or separately at the discretion of a physician.

In the following, the effects of specific compounds of the present disclosure are described in detail with reference to examples.

## Examples

### Example 1: General Method for Synthesizing Compound 537 (TDM-181137)

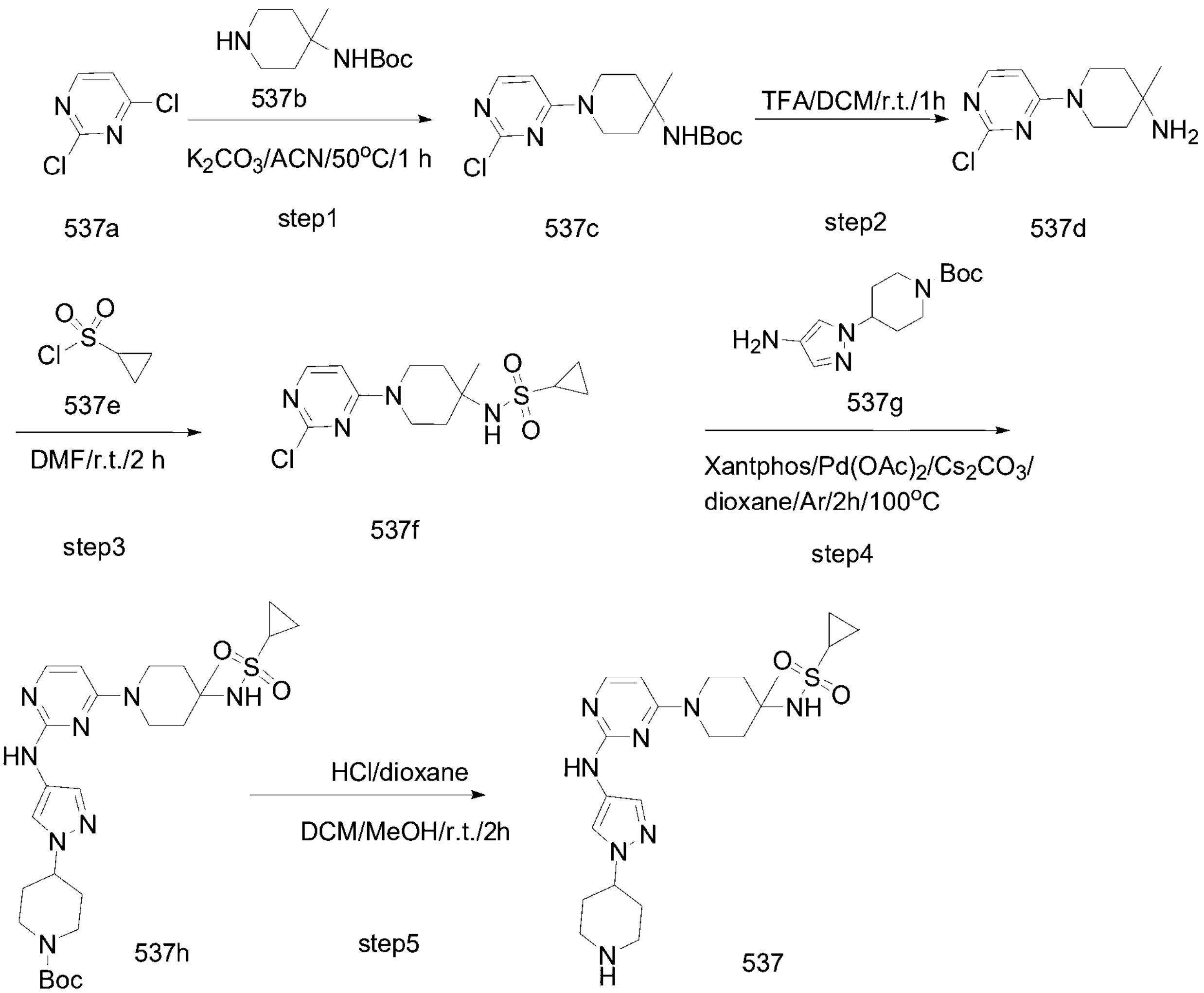


### Step 1: Compound 537c

### Tert-butyl 1-(2-chloropyrimidin-4-yl)-4-methylpiperidin-4-carbamate

Potassium carbonate (555.60 mg, 4.02 mmol) was added to a solution of compound 537a (300 mg, 2.01 mmol) and compound 537b (430.74 mg, 2.01 mmol) in acetonitrile (30 mL), and the reaction solution was stirred at 50°C for 1 hour. After the reaction completed, the mixture was concentrated under reduced pressure, water (60 mL) was added to the residue, the mixture was extracted with ethyl acetate (30 mL×3), and the organic layers were combined, washed with brine (50 mL), and dried over sodium sulfate. The filtrate was concentrated under reduced pressure, and the residue was purified by silica gel chromatography [petroleum ether/ethyl acetate = 0-24/76] to obtain the compound (compound 537c, 548.3 mg, yield: 83.5%) as a colorless solid. LCMS $[M+1]^+$ = 327.1.

### Step 2: Compound 537d

**4-Chloropyrimidin-4-chloropyridin-4-amine**

Trifluoroacetic acid (2.49 mL) was added to a solution of compound 537c (548.3 mg, 1.68 mmol) in dichloromethane (22 mL). The reaction solution was stirred for 1 hour at room temperature. After the reaction completed, the mixture was concentrated under reduced pressure, water (60 mL) was added to the residue, the mixture was basified with a potassium carbonate solution and extracted with ethyl acetate (50 mL×3), and the organic layers were combined, washed with brine (50 mL), and dried over sodium sulfate. The filtrate was concentrated under reduced pressure to obtain a brown compound (compound 537d, 337.2 mg, yield: 88.5%) and the crude product was used directly for subsequent steps. LCMS $[M+1]^+$ = 227.1.

**Step 3: Compound 537f**

**N-(1-(2-chloropyrimidin-4-yl)-4-methylpiperidin-4-yl)cyclopropanesulfonamide**

Triethylamine (0.62 mL, 4.46 mmol) and compound 537e (0.21 mL, 2.23 mmol) were added to a solution of compound 537d (337.2 mg, 1.49 mmol) in N,N-dimethylformamide (15 mL), and the mixture was stirred for 2 hours at room temperature. After the reaction completed, the mixture was concentrated under reduced pressure, water (60 mL) was added to the residue, the mixture was extracted with ethyl acetate (40 mL×3), and the organic layers were combined, washed with brine (50 mL), and dried over sodium sulfate. The filtrate was concentrated under reduced pressure, and the residue was purified by silica gel chromatography [petroleum ether/ethyl acetate = 0-44/56] to obtain the compound (compound 537f, 231.8 mg, yield: 47%) as a white solid. LCMS $[M+1]^+$ = 331.1.

**Step 4: Compound 535h**

**Tert-butyl 4-(4-(4-(4-cyclopropanesulfonamido)-4-methylpiperidin-1-yl)pyrimidin-2-ylamino)-1H-pyrazol-1-yl) piperidine-1-carboxylate**

4,5-bis(diphenylphosphino)-9,9-dimethylxanthene (69.89 mg, 0.12 mmol), palladium acetate (13.56 mg, 0.06 mmol) and cesium carbonate (195.49 mg, 0.6 mmol) were added to a solution of compound 537f (100 mg, 0.3 mmol) and compound 537g (96.6 mg, 0.36 mmol) in A soluiton of HCl in dioxane (5 mL), and the mixture was degassed under vacuum and subjected to argon displacement. The reaction solution was stirred for 2 hours at 100°C. After the reaction completed, the mixture was concentrated under reduced pressure, and the residue was purified by silica gel chromatography (dichloromethane: dichloromethane containing 10% methanol = 0-60/40) to obtain the product (compound 537h, 106.4 mg, yield: 63.7%) as a brown solid. LCMS $[M+1]^+$= 561.2.

**Step 5: Compound 535**

**N-(4-methyl-1-(2-(1-(4-piperidinyl)-1H-pyrazol-4-ylamino)pyrimidin-4-ylpiperidin-4-yl)cyclopropanesulfonamide**

A soluiton of HCl in dioxane (0.89 mL) was added to a solution A (dichloromethane: methanol = 10:1) of compound 537h (100 mg, 0.178 mmol). The reaction solution was stirred for 2 hours at room temperature. After the reaction ended, the mixture was concentrated under reduced pressure, and the residue was purified via preparative HPLC (formic acid), to obtain the compound (compound 537, 18.9 mg, yield: 23%) as a white solid. LCMS $[M+1]^+$ = 461.2.

$^1$H NMR (400 MHz, MeOD) δ 7.93 (s, 1H), 7.66 (s, 2H), 6.58 (d, J = 7.3 Hz, 1H), 4.59 (s, 2H), 3.91 (s, 1H), 3.66 (s, 2H), 3.57 (d, J = 12.7 Hz, 2H), 3.23 (t, J = 10.8 Hz, 2H), 2.63 (dq, J = 7.9, 4.9 Hz, 1H), 2.33 (s, 4H), 2.19 (d, J = 13.9 Hz, 2H), 1.65 (t, J = 10.6 Hz, 2H), 1.52 (s, 3H), 1.15 - 1.10 (m, 2H), 1.09 - 1.03 (m, 2H).

**Example 2: General Method for Synthesizing Compound 563 (TDM-181163)**

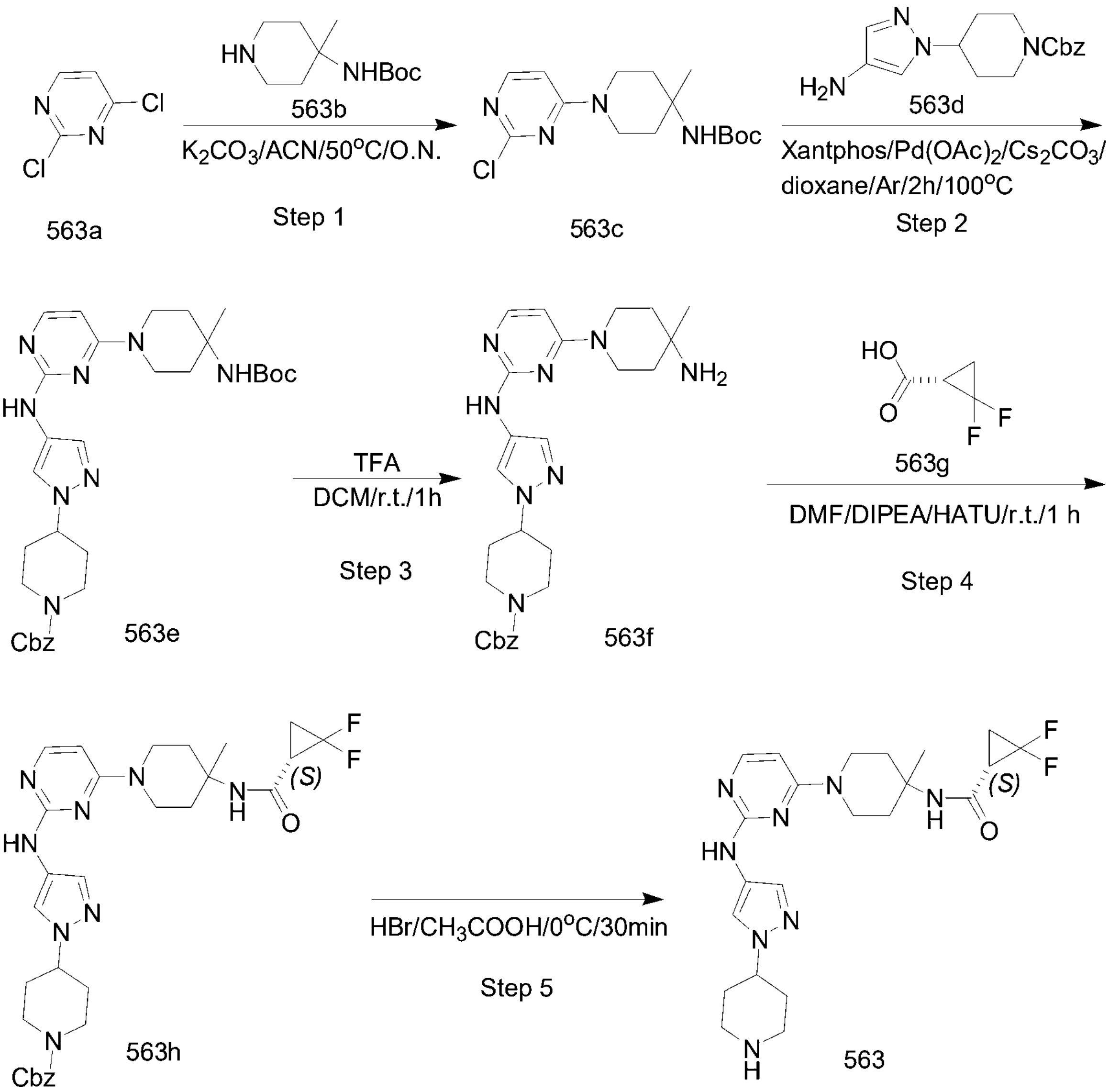


**Step 1: Compound 563c**

**Tert-butyl (1-(2-chloropyrimidin-4-yl)-4-methylpiperidin-4-yl)carbamate**

Potassium carbonate (557 mg, 4.028 mmol) was added to a solution of compound 563a (300 mg, 2.014 mmol) and compound 563b (453 mg, 2.115 mmol) in acetonitrile (30 mL), and the mixture was heated to 50°C and stirred overnight. The mixture was added to water (150 mL), the mixture was extracted with ethyl acetate (50 mL×3), organic layers were combined, rinsed with brine, and dried over sodium sulfate, the filtrate was concentrated under reduced pressure, and the residue was purified by silica gel chromatography (ethyl acetate/petroleum ether = 0-45/55), to obtain the product (compound 563c, 528.5 mg, yield: 80.3%) as a white solid. LCMS $[M+1]^+$ = 327.

**Step 2: Compound 563e**

**Benzyl 4-(4-((4-(4-(tert-butoxycarbonyl)amino)-4-methylpiperidin-1-yl)pyrimidin-2-yl)amino)-1H-pyrazol-1-yl) piperidine-1-carboxylate**

1,4-dioxane (5 mL) was added to a mixture of compound 563c (109 mg, 0.333 mmol), compound 563d (100 mg, 0.333 mmol), palladium acetate (7.5 mg, 0.033 mmol), xantphos (38 mg, 0.067 mmol), and cesium carbonate (217 mg, 0.666 mmol). The mixture was degassed under vacuum, argon displacement was employed several times, and then the mixture was heated to 100°C and stirred for 2 hours. The mixture was concentrated under reduced pressure, the residue

was purified via silica gel chromatography (dichloromethane containing 10% methanol: dichloromethane = 0-62/38), to obtain the product (compound 563e, 131.3 mg, yield: 66.7%) as a white solid. LCMS $[M+1]^+$ = 591.

**Step 3: Compound 563f**

**Benzyl 4-(4-((4-(4-amino-4-methylpiperidin-1-yl)pyrimidin-2-yl)amino)-1H-pyrazol-1-yl)piperidine-1-carboxylate**

Trifluoroacetic acid (0.7 mL) was added to a solution of compound 563e (90 mg, 0.152 mmol) in dichloromethane (9 mL) at room temperature. The mixture was stirred for 1 hour. The mixture was then concentrated under reduced pressure, the residue was added to water and neutralized with an aqueous solution of sodium carbonate, the mixture was extracted with ethyl acetate (20 mL×3), organic layers were combined and rinsed with brine, the filtrate was concentrated under reduced pressure, to obtain the product (compound 563f, 73.5 mg, yield: 98.5%) as a yellow solid. LCMS $[M+1]^+$ = 491.

**Step 4: Compound 563h**

**Benzyl (S)-4-(4-((4-(4-(2,2-difluorocyclopropane-1-carboxamido)-4-methylpiperidin-1-yl)pyrimidin-2-yl)amino)-1H-pyrazol-1-yl)piperidine-1-carboxylate**

2-(7-azabenzotriazol-1-yl)-N,N,N',N'-tetramethyluronium hexafluorophosphate (85 mg, 0.22 mmol) and compound 563g (27.5 mg, 0.225 mmol) were added to a solution of compound 563f (73.5 mg, 0.15 mmol) and N,N-diisopropylethylamine (29 mg, 0.225 mmol) in N,N-dimethylformamide (5 mL) at room temperature. The mixture was stirred for 1 hour. The mixture was then added to water and extracted with ethyl acetate (30 mL×3), organic layers were combined and rinsed with brine, the filtrate was concentrated under reduced pressure, the residue was purified via silica gel chromatography (dichloromethane containing 10% methanol: dichloromethane = 0-70/30), to obtain the product (compound 563h, 80 mg, yield: 61.1%) as a yellow solid. LCMS $[M+1]^+$ = 595.

**Step 5: Compound 563**

**(S)-2,2-Difluoro-N-(4-methyl-1-(2-((1-(piperidin-4-yl)-1H-pyrazol-4-yl)amino)pyrimidin-4-yl)piperidin-4-ylcyclopropane-1-carboxamide**

The compound 563h (70 mg, 0.118 mmol) was dissolved in $HBr/CH_3COOH$ (2 mL) at 0°C and the mixture was stirred for 30 minutes at 0°C. The mixture was then concentrated under reduced pressure, and the residue was purified via preparative HPLC (formic acid), to obtain the product (compound 563, TDM-181163, 5.9 mg, yield: 10.9%) as a white solid. LCMS $[M+1]^+$ = 486.3.

$^1H$ NMR (400 MHz, DMSO) δ 8.84 (s, 1H), 8.18 (s, 1H), 7.99 (s, 1H), 7.89 (d, J = 6.0 Hz, 1H), 7.80 (s, 1H), 7.51 (s, 1H), 6.19 (d, J = 6.1 Hz, 1H), 4.39 (dt, J = 14.9, 5.4 Hz, 1H), 3.90 (s, 2H), 3.36 (d, J = 12.8 Hz, 2H), 3.26 (s, 3H), 3.05 - 2.97 (m, 2H), 2.67 (ddd, J = 14.7, 10.9, 8.0 Hz, 1H), 2.13 (d, J = 13.7 Hz, 4H), 2.09 - 1.97 (m, 2H), 1.90 - 1.72 (m, 2H), 1.49 (d, J = 11.1 Hz, 2H), 1.32 (s, 3H).

**Example 3: General Method for Synthesizing Compound 564 (TDM-181164)**

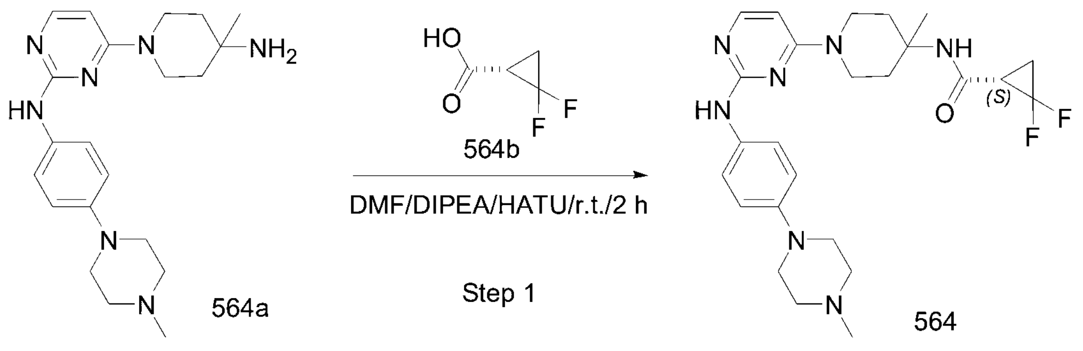


**Step 1: Compound 564**

**(S)-2,2-Difluoro-N-(4-methyl-1-(2-((4-(4-methylpiperazin-1-yl)phenyl)amino)pyrimidin-4-yl)piperidin-4-yl)cyclopropane-1-carboxamide**

2-(7-azabenzotriazol-1-yl)-N,N,N',N'-tetramethyluronium hexafluorophosphate (44.8 mg, 0.118 mmol) and compound 564b (19.2 mg, 0.157 mmol) were added to a solution of compound 564a (30 mg, 0.079 mmol) and N,N-diisopropylethylamine (15.2 mg, 0.118 mmol) in N,N-dimethylformamide (3 mL) at room temperature. The mixture was stirred for 2 hours at room temperature. The mixture was concentrated under reduced pressure, and the residue was purified via silica gel chromatography (100% methanol) and preparative HPLC (formic acid), to obtain the product (compound 564, 6.2 mg, yield: 16.2%) as a yellow solid. LCMS $[M+1]^+$ = 486.3.

$^1$H NMR (400 MHz, DMSO) δ8.75 (s, 1H), 8.16 (s, 2H), 7.97 (s, 1H), 7.89 (d, J = 6.0 Hz, 1H), 7.53 (d, J = 9.0 Hz, 2H), 6.84 (d, J = 9.1 Hz, 2H), 6.21 (d, J = 6.1 Hz, 1H), 3.92 (s, 2H), 3.23 (dt, J = 13.7, 9.0 Hz, 2H), 3.10 - 3.02 (m, 4H), 2.66 (ddd, J = 14.4, 11.0, 8.1 Hz, 1H), 2.57 - 2.52 (m, 4H), 2.28 (s, 3H), 2.13 (d, J = 10.6 Hz, 2H), 1.91-1.71 (m, 2H), 1.46 (td, J = 10.5, 4.0 Hz, 2H), 1.31 (s, 3H).

**Example 4: General Method for Synthesizing Compound 565 (TDM-181165)**

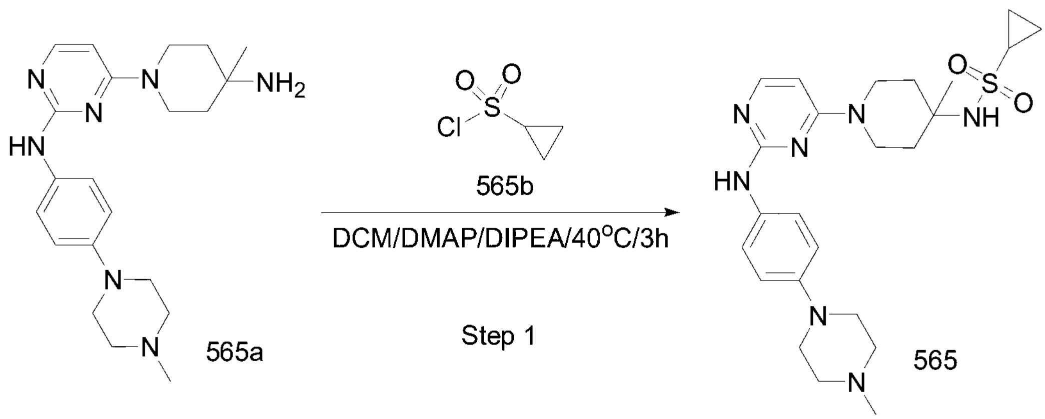


**Step 1: Compound 565**

**N-(4-methyl-1-(2-((4-(4-methylpiperazin-1-yl)phenyl)amino)pyrimidin-4-yl)piperidin-4-yl)cyclopropanesulfonamide**

4-dimethylaminopyridine (9.6 mg, 0.079 mmol) and compound 565b (19.2 mg, 0.157 mmol) were added to a solution of compound 565a (30 mg, 0.079 mmol) and N,N-diisopropylethylamine (15.2 mg, 0.118 mmol) in dichloromethane (2 mL) at room temperature. The mixture was heated to 40°C and stirred for 3 hours. The mixture was concentrated under reduced pressure, and the residue was purified via silica gel chromatography (dichloromethane containing 10% methanol: dichloromethane = 0-75/25) and preparative HPLC (formic acid), to obtain the product (compound 565, 13.3 mg, yield: 34.7%) as a white solid. LCMS $[M+1]^+$ = 486.3.

$^1$H NMR (400 MHz, DMSO) δ 8.74 (s, 1H), 8.14 (s, 2H), 7.89 (d, J = 6.1 Hz, 1H), 7.53 (d, J = 9.0 Hz, 2H), 6.86 (d, J = 4.1 Hz, 2H), 6.84 (s, 1H), 6.21 (d, J = 6.1 Hz, 1H), 3.79 (s, 2H), 3.51 - 3.44 (m, 2H), 3.07 (s, 4H), 2.65 - 2.60 (m, 1H), 2.57 (s, 4H), 2.30 (s, 3H), 1.93 (d, J = 14.1 Hz, 2H), 1.57 - 1.46 (m, 2H), 1.40 (s, 3H), 0.97 (d, J = 3.4 Hz, 2H), 0.95 (s, 2H).

**Example 5: General Method for Synthesizing Compound 572 (TDM-181172)**

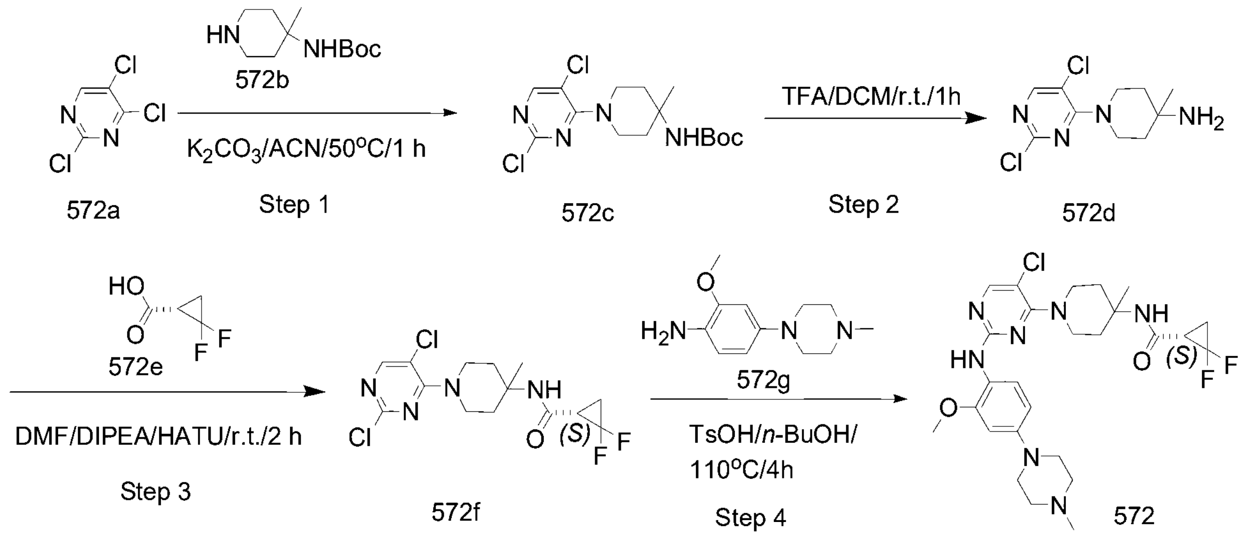


**Step 1: Compound 572c**

**(1-(2,5-Dichloropyrimidin-4-yl)-4-methylpiperidin-4-yl)carbamate**

Potassium carbonate (1.5 g, 10.9 mmol) was added to a solution of compound 572a (1 g, 5.45 mmol) and compound 572b (1.17 g, 5.45 mmol) in acetonitrile (50 mL). The reaction solution was stirred at 50°C for 1 hour, and the product was detected by LCMS $[M+1]^+ = 361.2$. The mixture was added into water (50 mL) and extracted with ethyl acetate (50 mL×2). The organic phases were combined, washed with brine, dried over anhydrous $Na_2SO_4$, and concentrated under reduced pressure. The organic layer was concentrated and purified by flash chromatography (40 g column, 35 minutes), eluting with PE/EA = 0-60%, to obtain compound 572c (1.65 g, yield: 84%) as a white solid. LCMS$[M+1]^+$=361.2.

**Step 2: Compound 572d**

**1-(2,5-Dichloropyrimidin-4-yl)-4-methylpiperidin-4-amine**

Trifluoroacetic acid (10 mL, 134.62 mmol) was added to a solution of compound 572c (1.1 g, 3.05 mmol) in dichloromethane (100 mL) at 0°C. The mixture was then stirred at room temperature for 1 hour, and the product was detected by LCMS $[M+1]^+ = 261.1$. The mixture was added into water (60 mL) and extracted with ethyl acetate (60 mL×2), and the organic layers were combined. The combined organic phases were washed with brine, dried over anhydrous sodium sulfate, and concentrated under reduced pressure. The organic layer was concentrated and purified by flash chromatography (40 g column, 35 minutes), eluting with DCM/(DCM:MeOH = 10:1) = 0 to 50%, to obtain compound 572d (870 mg, crude product) as a yellow solid. LCMS$[M+1]^+$ = 261.1.

**Step 3: Compound 572f**

**(S)-N-(1-(2,5-dichloropyrimidin-4-yl)-4-methylpiperidin-4-yl)-2,2-difluorocyclopropane-1-carboxamide**

A solution of compound 572d (350 mg, 1.35 mmol), compound 572e (246 mg, 2.02 mmol), and 2-(7-azabenzotriazol-1-yl)-N,N,N',N'-tetramethyluronium hexafluorophosphate (767 mg, 2.05 mmol) in DMF (25 mL) was added to a 50 mL flask, N,N-diisopropylethylamine (261 mg, 2.01 mmol) was added, and the mixture was stirred for 1 hour at room temperature. The product was detected by LCMS$[M+1]^+$=365.1. The combined organic phase was rinsed with brine, dried over anhydrous sodium sulfate, and concentrated under reduced pressure. The organic layer was concentrated, purified by flash chromatography (40 g of column, 35 minutes), and eluted with PE/EA=0-75%, to obtain a yellow oily liquid (compound 572f, 670 mg, crude product) as a product. LCMS[M+1]+=365.1.

**Step 4: Compound 572**

**(S)-N-(1-(2,5-dichloropyrimidin-4-yl)-4-methylpiperidin-4-yl)-2,2-difluorocyclopropane-1-carboxamide**

Compound 572f (74.5 mg, 0.2 mmol), compound 572g (45 mg, 0.2 mmol), and p-toluenesulfonic acid (77 mg,

0.407 mmol) were added in a 50 mL beaker and dissolved in n-butanol (4 mL), and the mixture was stirred for 4 hours at 110°C. LCMS[M+1]+=550.3, and a product was detected. The mixture was added to water (20 mL) and extracted with ethyl acetate (20 mL×2), and organic layers were combined. The organic phases were rinsed with brine, dried over anhydrous sodium sulfate, and concentrated under reduced pressure. The crude product was purified via preparative HPLC, to obtain the product (compound 572, 5.3 mg, yield: 13%) as a white solid. LCMS [M+1]+= 550.3.

$^1$H NMR (401 MHz, DMSO) δ 8.20 (s, 1H), 7.98 (s, 1H), 7.95 (s, 1H), 7.72 - 7.67 (m, 2H), 6.61 (d, *J* = 2.5 Hz, 1H), 6.45 (dd, *J* = 8.9, 2.5 Hz, 1H), 3.84 (d, *J* = 14.4 Hz, 2H), 3.80 (s, 3H), 3.26 - 3.20 (m, 2H), 3.14 - 3.08 (m, 4H), 2.71 - 2.61 (m, 1H), 2.47 - 2.42 (m, 4H), 2.23 (s, 3H), 2.15 (d, *J* = 13.0 Hz, 2H), 1.88 - 1.72 (m, 2H), 1.56 (dd, *J* = 13.8, 10.4 Hz, 2H), 1.32 (s, 3H).

N-(1-(5-chloro-2-((4-(4-methylpiperazin-1-yl)phenyl)amino)pyrimidin-4-yl)-4-methylpiperidin-4-yl)cyclopropanesulfonamide (TDM-181173) (white solid, 52.2 mg, yield: 37.17%) and (S)-N-(1-(5-chloro-2-((4-(4-methylpiperazin-1-yl)phenyl)amino)pyrimidin-4-yl)-4-methylpiperidin-4-yl)-2,2-difluorocyclopropanecarboxamide (TDM-181174) (white solid, 5 mg, yield: 60%) were synthesized in a method similar to that in Example 5.

| TDM NO. | Structure | LCMS [M+1]+ | $^1$H-NMR |
|---|---|---|---|
| TDM-181173 | | 520.3 | $^1$H NMR (400 MHz, MeOD) δ 8.34 (s, 1H), 7.91 (s, 1H), 7.50 (d, J = 8.9 Hz, 2H), 6.97 (d, J = 8.9 Hz, 2H), 3.96 (d, J = 14.1 Hz, 2H), 3.57 (t, J = 11.8 Hz, 2H), 3.29-3.25 (m, 2H), 3.18 (d, J = 43.7 Hz, 5H), 2.78 (d, J = 20.6 Hz, 4H), 2.62-2.55 (m, 1H), 2.08 (d, J = 14.1 Hz, 2H), 1.70 (t, J = 10.1 Hz, 2H), 1.50 (s, 3H), 1.09 (d, J = 4.3 Hz, 2H), 1.05-0.98 (m, 2H). |
| TDM-181174 | | 520.3 | $^1$H NMR (401 MHz, DMSO) δ 9.12 (s, 1H), 8.20 (s, 1H), 8.01 (s, 1H), 7.97 (s, 1H), 7.50 (d, J = 9.0 Hz, 2H), 6.85 (d, J = 9.1 Hz, 2H), 3.86 (dt, J = 19.7, 7.7 Hz, 2H), 3.28 (s, 3H), 3.07-3.02 (m, 4H), 2.72-2.60 (m, 2H), 2.46-2.42 (m, 5H), 2.22 (s, 3H), 2.17 (d, J = 13.3 Hz, 2H), 1.93-1.70 (m, 2H), 1.65-1.50 (m, 2H), 1.33 (s, 3H). |

**Example 6: General Method for Synthesizing Compound 575 (TDM-181175)**

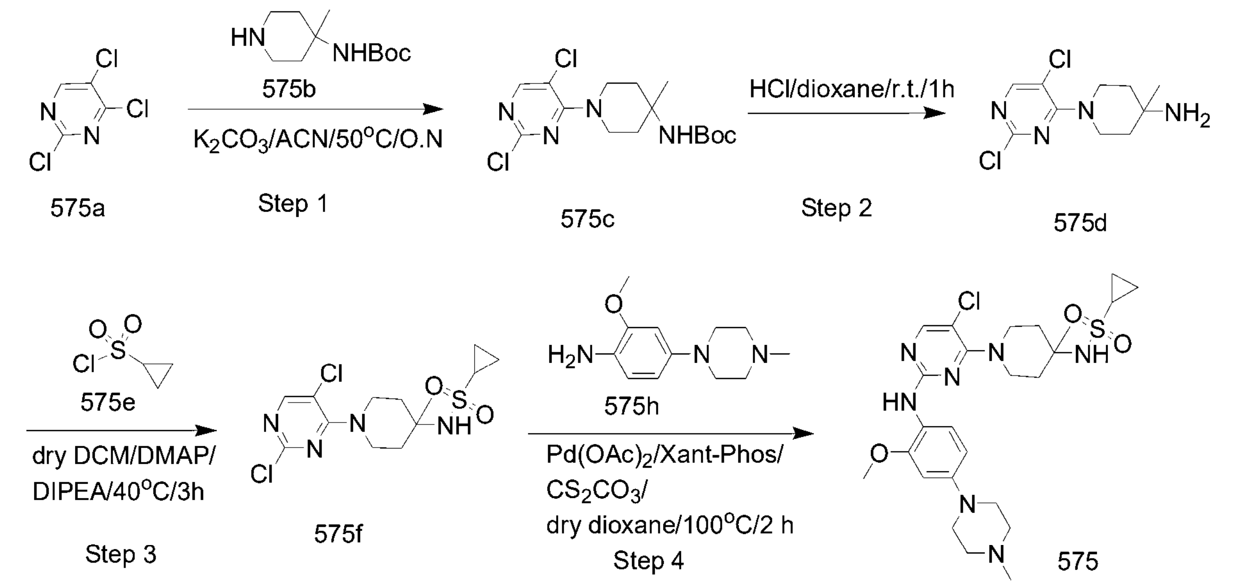

575a 575c 575d 575f 575

**Step 1: Compound 575c**

**(1-(2,5-Dichloropyrimidin-4-yl)-4-methylpiperidin-4-yl)carbamate**

Potassium carbonate (1.5 g, 10.9 mmol) was added to an acetonitrile solution (50 mL) containing compound

575a (1 g, 5.45 mmol) and compound 575b (1.17 g, 5.45 mmol). The reaction solution was stirred overnight at 50°C, and the product was detected by LCMS $[M+1]^+$ = 361.2. The mixture was added to water (50 mL) and extracted with ethyl acetate (50 mL×2), organic phases were combined, the combined organic phases were rinsed with brine and dried over anhydrous $Na_2SO_4$, and the mixture was concentrated under reduced pressure. The organic layer was concentrated, purified by flash chromatography (40 g of column, 35 minutes), and eluted with PE/EA = 0-60%, to obtain the product (compound 575c, 1.65 g, yield: 84%) as a white solid. LCMS$[M+1]^+$=361.2.

**Step 2: Compound 575d**

**1-(2,5-Dichloropyrimidin-4-yl)-4-methylpiperidin-4-amine**

A soluiton of HCl in dioxane (10 mL, 134.62 mmol) was added to a solution of compound 575c (1.1 g, 3.05 mmol) in 1,4-dioxane (100 mL) at 0°C. The mixture was then stirred for 1 hour at room temperature, and the product was detected by LCMS $[M+1]^+$ = 261.1. The mixture was added to water (60 mL) and extracted with ethyl acetate (60 mL×2), and organic layers were combined. The organic phases were rinsed with brine, combined, dried over anhydrous sodium sulfate, and concentrated under reduced pressure. The organic layer was concentrated, purified by flash chromatography (40 g of column, 35 minutes), and eluted with DCM/(DCM: MeOH = 10:1) = 0-50%, to obtain a yellow solid (compound 575d, 870 mg, crude product) as a product. LCMS$[M+1]^+$ = 261.1.

**Step 3: Compound 575f**

**N-(1-(2,5-dichloropyrimidin-4-yl)-4-methylpiperidin-4-yl)cyclopropanesulfonamide**

N,N-diisopropylethylamine (321.21 mL, 2.49 mmol) was added to a solution of compound 575d (430.9 g, 1.66 mmol) in anhydrous dichloromethane (20 mL), and 4-dimethylaminopyridine (202.43 mg, 1.66 mmol) and compound 575e (349.40 mg, 2.49 mmol) was then added at 0°C. The reaction solution was heated to 40°C and stirred for 3 hours. LCMS $[M+1]^+$=365, and it was detected that the reaction ended. Post-treatment: The reaction solution was added into water, an aqueous phase was extracted with dichloromethane (40 mL×2), the organic phases were combined and rinsed with saturated brine, dried with anhydrous sodium sulfate, concentrated and dried, and a resulting crude product was subjected to column chromatography [eluent: EA/PE = 0%-30%], to obtain the target compound (compound 575f, 554.9 mg, yield: 82%) as a yellow liquid, where LCMS $[M+1]^+$ =365.

**Step 4: Compound 575**

**N-(1-(5-chloro-2-((2-methoxy-4-(4-methylpiperazin-1-yl)phenyl)amino)pyrimidin-4-yl)4-methylpiperidin-4-yl)cyclopropanesulfonamide**

Compound 575h (109.05 mg, 0.49 mmol), palladium acetate (18.41 mg, 0.082 mmol), 4,5-bis(diphenylphosphine)-9,9-dimethylxanthene (94.89 mg, 0.164 mmol) and cesium carbonate (267.16 mg, 0.82 mmol) were added to compound 575f (150 mg, 0.41 mmol). The reaction solution was subjected to argon displacement several times, and then anhydrous 1,4-dioxane (10 mL) was added. The reaction solution was subjected to argon displacement three times. The reaction solution was heated to 100°C and stirred for two hours. LCMS$[M+1]^+$=550.2, and it was detected that the reaction ended. Post-treatment: The reaction solution was concentrated and dried, and a resulting crude product was subjected to column chromatography [eluent: (dichloromethane: methanol=10:1)/DCM = 0%-95%]. The crude product was then purified via preparative HPLC to obtain the target compound (compound 575, 75.7 mg, yield: 33.56%) as a white solid, LCMS $[M+1]^+$ = 550.2.

$^1$H NMR (400 MHz, MeOD) δ 8.44 (s, 1H), 8.01 (d, *J* = 8.8 Hz, 1H), 7.91 (d, *J* = 1.7 Hz, 1H), 6.68 (d, *J* = 2.3 Hz, 1H), 6.56 (dd, *J* = 8.8, 2.3 Hz, 1H), 3.97 (d, *J* = 13.1 Hz, 2H), 3.89 (s, 3H), 3.58 (t, *J* = 11.8 Hz, 2H), 3.28 (d, *J* = 5.3 Hz, 4H), 3.03 (d, *J* = 4.7 Hz, 4H), 2.66 (d, *J* = 2.7 Hz, 3H), 2.63 - 2.54 (m, 1H), 2.09 (d, *J*= 13.9 Hz, 2H), 1.74 - 1.65 (m, 2H), 1.50 (s, 3H), 1.13 - 1.07 (m, 2H), 1.06 - 0.98 (m, 2H).

**Example 7: General Method for Synthesizing Compound 578 (TDM-181178)**

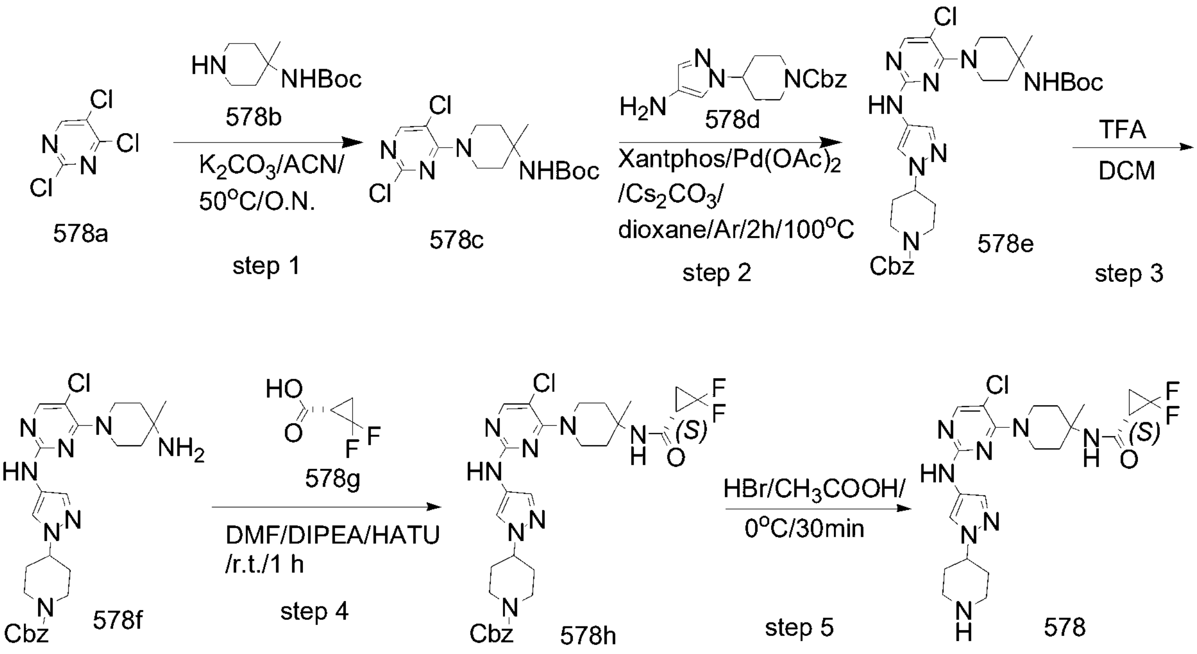


**Step 1: Compound 578c**

**(1-(2,5-Dichloropyrimidin-4-yl)-4-methylpiperidin-4-yl)carbamate**

Potassium carbonate (1.5 g, 10.9 mmol) was added to a solution of compound 578a (1 g, 5.45 mmol) and compound 578b (1.17 g, 5.45 mmol) in acetonitrile (50 mL). The reaction mixture was stirred at 50°C overnight, and the product was detected by LCMS $[M+1]^+ = 361.2$. The mixture was added into water (50 mL) and extracted with ethyl acetate (50 mL×2). The organic phases were combined, washed with brine, dried over anhydrous $Na_2SO_4$, and concentrated under reduced pressure. The concentrate was purified by flash chromatography (40 g column, 35 minutes), eluting with PE/EA = 0-60%, to obtain the product (compound 578c, 1.65 g, yield: 84%) as a white solid. LCMS$[M+1]^+$=361.2.

**Step 2: Compound 578e**

**Benzyl 4-(4-((4-(4-(tert-butoxycarbonyl)amino)-4-methylpiperidin-1-yl)-5-chloropyrimidin-2-yl)amino)-1H-pyrazol-1-yl)piperidine-1-carboxylate**

Compound 578c (251 mg, 0.7 mmol), compound 578d (210 mg, 0.7 mmol), palladium acetate (31.3 mg, 0.14 mmol), 4,5-bis(diphenylphosphino)-9,9-dimethylxanthene (161.26 mg, 0.279 mmol) and cesium carbonate (454 mg, 1.4 mmol) were dissolved in super dry 1,4-dioxane (25 mL) in a 50 mL flask. The mixture was stirred at 100°C under argon for 2 hours. The reaction was monitored by TLC and LCMS, and the product was confirmed by LCMS [M+1]+ = 625.3. The mixture was added into water (50 mL) and extracted with ethyl acetate (50 mL×2). The organic layers were combined, washed with brine, dried over anhydrous sodium sulfate, and concentrated under reduced pressure. The concentrate was purified by flash chromatography (12 g column, 25 minutes), eluting with DCM/(DCM:MeOH = 10:1) = 0-50%, to obtain the product (compound 578e, 240 mg, yield: 50%) as a white solid. LCMS$[M+1]^+$=625.3.

**Step 3: Compound 578f**

**Benzyl 4-(4-((4-(4-amino-4-methylpiperidin-1-yl)-5-chloropyrimidin-2-yl)amino)-1H-pyrazol-1-yl)piperidine-1-carboxylate**

Compound 578e (100 mg, 0.16 mmol) was dissolved in dichloromethane (10 mL) at 0°C, and trifluoroacetic acid (1 mL) was added. The mixture was stirred at room temperature for 1 hour. The reaction was monitored by TLC and LCMS, and the product was detected by LCMS [M+1]+ = 525.3. The reaction mixture was concentrated under reduced pressure, added into water (30 mL), and adjusted to pH > 8 with 50% potassium carbonate solution. The mixture was extracted with ethyl acetate (30 mL×2). The organic layers were combined, washed with brine, dried over anhydrous sodium sulfate, and concentrated under reduced pressure to obtain the product (compound 578f, 170 mg, crude) as a yellow solid. LCMS[M+1]$^+$= 525.3.

**Step 4: Compound 578h**

**Benzyl (S)-4-(4-((5-chloro-4-(2-(2,2-difluorocyclopropane-1-carboxamido)-4-methylpiperidin-1-yl)pyrimidin-2-yl)amino)-1H-pyrazol-1-yl)piperidine-1-carboxylate**

Compound 578f (140 mg, 0.27 mmol), compound 578g (48.8 mg, 0.4 mmol) and 2-(7-azabenzotriazol-1-yl)-N,N,N',N'-tetramethyluronium hexafluorophosphate (152 mg, 0.4 mmol) were dissolved in N,N-dimethylformamide (15 mL) in a 50 mL flask, and N,N-diisopropylethylamine (51.6 mg, 0.4 mmol) was added. The mixture was stirred at room temperature for 1 hour. The reaction was monitored by TLC and LCMS, and the product was confirmed by LCMS [M+1]$^+$ = 629.2. The mixture was added into water (30 mL) and extracted with ethyl acetate (30 mL×2). The organic layers were combined, washed with brine, dried over anhydrous sodium sulfate, and concentrated under reduced pressure. The concentrate was purified by flash chromatography (12 g column, 25 minutes), eluting with DCM/(DCM:MeOH = 10:1) = 0-100%, to obtain the target product (compound 578h, 133 mg, yield: 74%) as a yellow oil. LCMS[M+1]$^+$=629.2.

**Step 5: Compound 578**

**(S)-2,2-Difluoro-N-(4-methyl-1-(5-methyl-2-((1-(piperidin-4-yl)-1H-pyrazol-4-yl)amino)pyrimidin-4-yl)piperidin-4-yl)cyclopropane-1-carboxamide**

Compound 578h (40 mg, 0.064 mmol) was added to a 25 mL flask and cooled at 0°C for a period, then a solution of hydrobromic acid in acetic acid (2 mL) was added dropwise. The mixture was stirred at 0°C for 0.5 hour. The reaction was monitored by TLC and LCMS, and the product was detected by LCMS [M+1]$^+$ = 495.2. The mixture was concentrated under reduced pressure. The crude product was purified by preparative HPLC to obtain the product (compound 578, 12 mg, yield: 37.5%) as a white solid. LCMS[M+1]$^+$=495.2.

$^1$H NMR (401 MHz, DMSO) δ 9.24 (s, 1H), 8.29 (s, 1H), 8.01 (s, 2H), 7.78 (s, 1H), 7.52 (s, 1H), 4.26 (s, 1H), 3.85 (d, *J* = 8.6 Hz, 2H), 3.31 (s, 2H), 3.20 (d, *J* = 12.0 Hz, 2H), 2.80 (t, *J* = 11.5 Hz, 2H), 2.72 - 2.63 (m, 1H), 2.19 (d, *J* = 11.3 Hz, 2H), 2.03 (d, *J* = 11.8 Hz, 2H), 1.97 - 1.64 (m, 5H), 1.60 (t, *J* = 11.9 Hz, 2H), 1.33 (s, 3H).

**Example 8: General Method for Synthesizing Compound 582 (TDM-181182)**

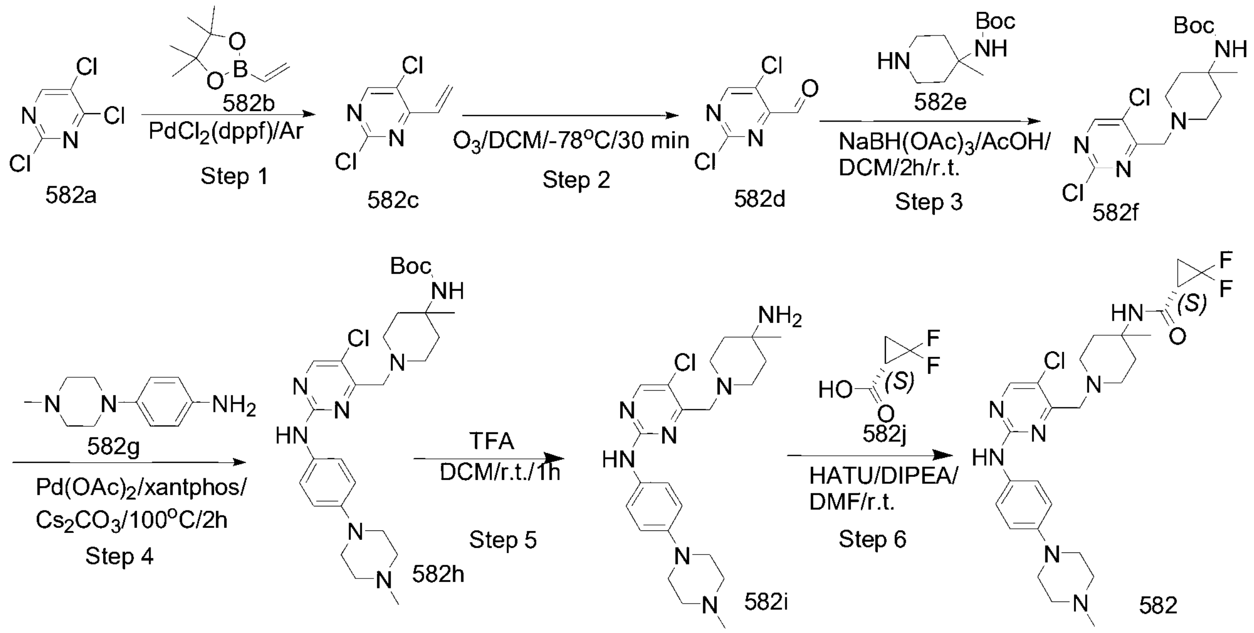


**Step 1: Compound 582c**

**2,5-Dichloro-4-vinylpyrimidine**

Compound 582b (1.69 g, 10.96 mmol), 1,1'-bis(diphenylphosphino)ferrocene dichloropalladium (800 mg, 1.1 mmol), cesium carbonate (7.14 g, 21.92 mmol) and water (10 mL) were added to a solution of compound 582a (2.01 g, 10.96 mmol) in 1,4-dioxane (100 mL). The reaction mixture was degassed and backfilled with argon several times, then heated to 70°C and stirred for 1 hour. The reaction was confirmed complete. Post-treatment: The reaction mixture was added into water, and the aqueous phase was extracted with ethyl acetate (50 mL×3). The organic phases were combined, washed with saturated brine, dried over anhydrous sodium sulfate, and concentrated to dryness under reduced pressure. The crude product was purified by column chromatography [eluent: EA/PE = 0-5%] to obtain the target compound (compound 582c, 960 mg, yield: 47%) as a yellow oil. LCMS $[M+1]^+$=175, 177.

**Step 2: Compound 582d**

**2,5-Dichloropyrimidine-4-carbaldehyde**

Compound 582c (700 mg, 60% purity) was dissolved in dichloromethane (15 mL). The solution was cooled to -78°C and purged with ozone for 30 minutes. Then dimethyl sulfide was added, and the mixture was stirred for 3 minutes. The mixture was concentrated under reduced pressure, and the residue was purified by silica gel chromatography (ethyl acetate/petroleum ether = 0-30/70) to obtain the product (compound 582d, 323 mg, crude) as a white solid. LCMS $[M+1]^+$ = 177 & 179.

**Step 3: Compound 582f**

**Tert-butyl(1-((2,5-dichloropyrimidin-4-yl)methyl)-4-methylpiperidin-4-yl)carbamate**

Compound 582e (430 mg, 2.008 mmol) and acetic acid (2 drops) were added to a solution of compound 582d (323 mg, 1.825 mmol) in dichloromethane (18 mL) at room temperature. The mixture was stirred for 1 hour, then sodium triacetoxyborohydride (580 mg, 2.738 mmol) was added. The mixture was stirred for 20 minutes and concentrated under reduced pressure. The residue was purified by silica gel chromatography (DCM containing 10% MeOH/DCM = 0-15/85) to obtain the product (compound 582f, 350 mg, yield: 51.1%) as a white solid. LCMS $[M+1]^+$ = 375 & 377.

**Step 4: Compound 582h**

**Tert-butyl(1-((5-chloro-2-((4-(4-methylpiperazin-1-yl)phenyl)amino)pyrimidin-4-yl)methyl)-4-methylpiperidin-4-yl)carbamate**

1,4-Dioxane (10 mL) was added to a mixture of compound 582f (125 mg, 0.342 mmol), compound 582g (72 mg, 0.376 mmol), palladium acetate (15 mg, 0.0684 mmol), Xantphos (79 mg, 0.1318 mmol) and cesium carbonate (223 mg, 0.684 mmol) at room temperature. The mixture was heated to 100°C and stirred for 2 hours. The mixture was concentrated under reduced pressure, and the residue was purified by silica gel chromatography (DCM containing 10% MeOH/DCM = 0-80/20) to obtain the product (compound 582h, 125 mg, yield: 69.1%) as a white solid. LCMS $[M+1]^+$ = 530.

**Step 5: Compound 582i**

**4-((4-Amino-4-methylpiperidin-1-yl)methyl)-5-chloro-N-(4-(4-methylpiperazin-1-yl)phenyl)pyrimidin-2-amine**

Trifluoroacetic acid (1.5 mL) was added to a solution of compound 582h (125 mg, 0.236 mmol) in dichloromethane (10 mL) at room temperature. The mixture was stirred at room temperature for 1 hour. Water was added to the mixture, which was neutralized with aqueous potassium carbonate solution and extracted with ethyl acetate. The organic layers were combined, dried over sodium sulfate, and the filtrate was concentrated under reduced pressure. The residue was used directly in the next step. LCMS $[M+1]^+$ = 430.

**Step 6: Compound 582**

**(S)-N-(1-((5-chloro-2-((4-(4-methylpiperazin-1-yl)phenyl)amino)pyrimidin-4-yl)methyl)-4-methylpiperidin-4-yl)-2,2-difluorocyclopropane-1-carboxamide**

Compound 582j (21 mg, 0.174 mmol), 2-(7-azabenzotriazol-1-yl)-N,N,N',N'-tetramethyluronium hexafluorophosphate (66 mg, 0.174 mmol) and N,N-diisopropylethylamine (22.5 mg, 0.174 mmol) were added to a solution of compound 582i (50 mg, 0.116 mmol) in N,N-dimethylformamide (5 mL) at room temperature. The mixture was stirred for 1 hour. The mixture was concentrated under reduced pressure, and the residue was purified by silica gel chromatography (DCM containing 10% MeOH/DCM = 0-100/0) and preparative HPLC (formic acid) to obtain the product (compound 582, TDM-181182, 8.8 mg, yield: 14.2%) as a yellow solid. LCMS $[M+1]^+$ = 534.

$^1H$ NMR (400 MHz, DMSO) δ 10.44 (s, 1H), 9.74 (s, 1H), 8.49 (s, 1H), 8.14 (s, 1H), 7.57 (d, J = 7.9 Hz, 2H), 6.97 (d, J = 8.8 Hz, 2H), 4.57 (s, 2H), 3.68 (s, 4H), 3.45 (s, 2H), 2.99 (s, 6H), 2.82 (s, 3H), 2.65 (d, J = 12.9 Hz, 2H), 2.33 (s, 2H), 1.83 (d, J = 6.7 Hz, 4H), 1.31 (s, 3H).

### Example 9: General Method for Synthesizing Compound 583 (TDM-181183)

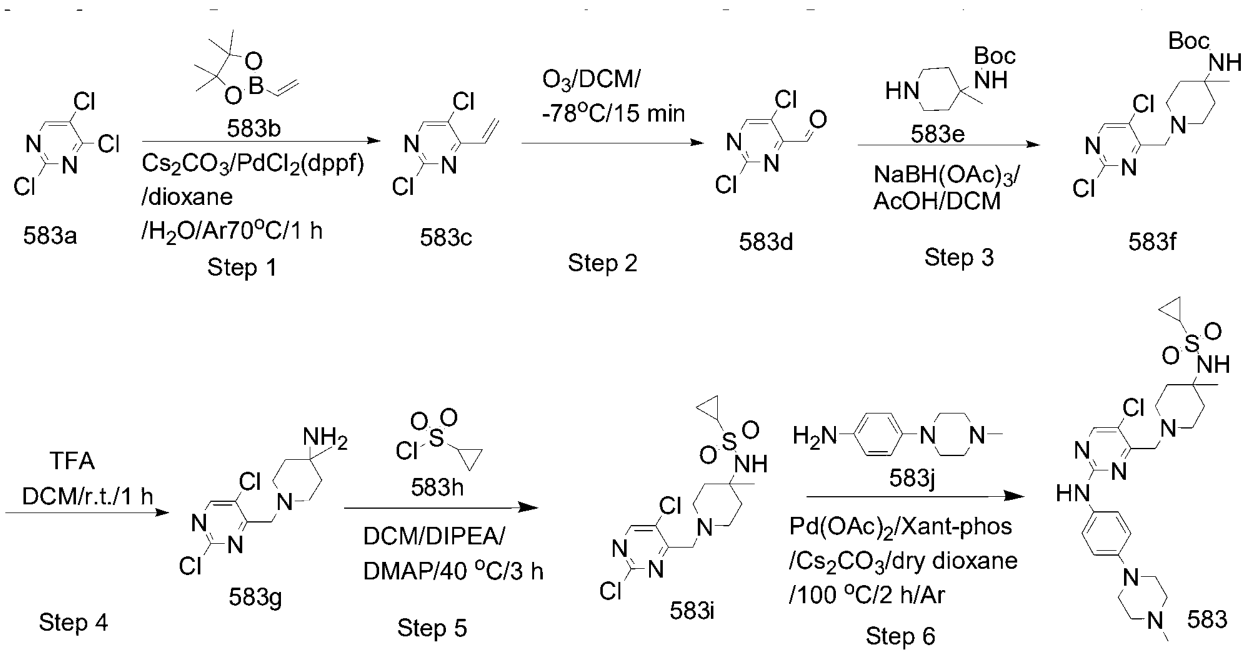


**Step 1: Compound 583c**

**2,5-Dichloro-4-vinylpyrimidine**

Compound 583b (1.69 g, 10.96 mmol), 1,1'-bis(diphenylphosphino)ferrocene dichloropalladium (800 mg, 1.1 mmol), cesium carbonate (7.14 g, 21.92 mmol) and water (10 mL) were added to a solution of compound 583a (2.01 g, 10.96 mmol) in 1,4-dioxane (100 mL). The reaction mixture was degassed and backfilled with argon several times, then heated to 70°C and stirred for 1 hour. The reaction was confirmed complete. Post-treatment: The reaction mixture was added into water, and the aqueous phase was extracted with ethyl acetate (50 mL×3). The organic phases were combined, washed with saturated brine, dried over anhydrous sodium sulfate, and concentrated to dryness under reduced pressure. The crude product was purified by column chromatography [eluent: EA/PE = 0-5%] to obtain the target compound (compound 583c, 960 mg, yield: 47%) as a yellow oil. LCMS $[M+1]^+$ =175, 177.

**Step 2: Compound 583d**

**2,5-Dichloropyrimidine-4-carbaldehyde**

A solution of compound 583c (960 mg, 5.48 mmol) in dichloromethane (130 mL) was added to a 100 mL three-necked flask. The reaction mixture was cooled to -78°C, and excess ozone was bubbled through the solution for 15 minutes. The reaction was confirmed complete. Post-treatment: Dimethyl sulfide was added dropwise at -78°C. The mixture was warmed to room temperature, stirred for 30 minutes, and concentrated to dryness under reduced pressure. The crude product was purified by column chromatography [eluent: EA/PE = 0-30%] to obtain the target compound (compound 583d, 90 mg, yield: 13.3%) as a yellow oil. LCMS $[M+1]^+$ =177, 179.

**Step 3: Compound 583f**

**Tert-butyl(1-((2,5-dichloropyrimidin-4-yl)methyl)-4-methylpiperidin-4-yl)carbamate**

Compound 583e (109 mg, 0.51 mmol) and acetic acid (1 drop) were added to a solution of compound 583d (90 mg, 0.51 mmol) in dichloromethane (10 mL). The reaction mixture was stirred at room temperature for 1.5 hours, then sodium triacetoxyborohydride (162.1 mg, 0.77 mmol) was added. The mixture was stirred for a further 30 minutes, and the reaction was confirmed complete. Post-treatment: The reaction mixture was added into water and extracted with ethyl acetate (50 mL×3). The organic phases were combined, washed with saturated brine, dried over anhydrous sodium sulfate, and concentrated to dryness under reduced pressure. The crude product was purified by column chromatography [eluent: EA/PE = 0-30%] to obtain the target compound (compound 583f, 76 mg, yield: 53%) as a yellow solid. LCMS $[M+1]^+$ =375, 377.

**Step 4: Compound 583g**

**1-((2,5-Dichloropyrimidin-4-yl)methyl)-4-methylpiperidin-4-amine**

Trifluoroacetic acid (1 mL) was added to a solution of compound 583f (100 mg, 0.27 mmol) in dichloromethane (10 mL). The reaction mixture was stirred at room temperature for 1 hour, and the reaction was confirmed complete. Post-treatment: The reaction mixture was added into water, and impurities were removed by extraction with ethyl acetate (50 mL×1). The aqueous phase was adjusted to pH 11 with potassium carbonate, then extracted with ethyl acetate (80 mL×3). The organic phases were combined, washed with saturated brine, dried over anhydrous sodium sulfate, and concentrated to dryness under reduced pressure to obtain the target compound (compound 583g, 86.5 mg, yield: 93.3%) as a yellow solid. LCMS $[M+1]^+$=275, 277.

**Step 5: Compound 583i**

**N-(1-((2,5-Dichloropyrimidin-4-yl)methyl)-4-methylpiperidin-4-yl)cyclopropanesulfonamide**

4-dimethylaminopyridine (38.4 mg, 0.31 mmol), N,N-diisopropylethylamine (81.3 mg, 0.63 mmol), and compound 583h (88.4 mg, 0.63 mmol) were added to a solution of compound 583g (86.5 mg, 0.31 mmol) in dichloromethane (8 mL). The reaction mixture was heated to 40°C and stirred for 3 hours, and the reaction was confirmed complete. Post-treatment: The reaction mixture was added into water and extracted with ethyl acetate (50 mL×3). The organic phases were combined, washed with saturated brine, dried over anhydrous sodium sulfate, and concentrated to dryness under reduced pressure. The crude product was purified by column chromatography [eluent: (DCM:MeOH = 10:1)/DCM = 0-20%] to obtain the target compound (compound 583i, 49.6 mg, yield: 34%) as a yellow solid. LCMS $[M+1]^+$ = 379, 381.

**Step 6: Compound 583**

**N-(1-((5-chloro-2-((4-(4-methylpiperazin-1-yl)phenyl)amino)pyrimidin-4-yl)methyl)-4-methylpiperidin-4-yl)cyclopropanesulfonamide**

Compound 583i (49.6 mg, 0.13 mmol), compound 583j (22.5 mg, 0.12 mmol), palladium acetate (5.8 mg, 0.03 mmol), 4,5-bis(diphenylphosphino)-9,9-dimethylxanthene (30 mg, 0.05 mmol), cesium carbonate (84.7 mg, 0.26 mmol) and 1,4-dioxane (5 mL) were added to a 100 mL three-necked flask. The reaction mixture was degassed and backfilled with argon several times, then heated to 100°C and stirred for 2 hours. The reaction was confirmed complete. Post-treatment: The reaction mixture was concentrated to dryness directly. The crude product was purified by column chromatography [eluent: (DCM:MeOH = 10:1)/DCM = 0-100%], and further purified by preparative HPLC to obtain the target compound (compound 583, 8.6 mg, yield: 12.4%) as a yellow solid. LCMS $[M+1]^+$ = 534.2.

$^1$H NMR (400 MHz, DMSO-d6) δ 9.57 (s, 1H), 8.38 (s, 1H), 8.16 (s, 2H), 7.58 (d, J = 8.9 Hz, 2H), 6.94 - 6.83 (m, 2H), 6.68 (s, 1H), 3.53 (s, 2H), 3.06 (t, J = 5.0 Hz, 4H), 2.65 - 2.58 (m, 2H), 2.58 - 2.52 (m, 4H), 2.47 (d, J = 4.7 Hz, 4H), 2.24 (s, 3H), 1.91 (d, J = 13.2 Hz, 2H), 1.53 (ddd, J = 12.9, 8.5, 3.6 Hz, 2H), 1.33 (s, 3H), 0.92 (d, J = 6.4 Hz, 4H).

N-(1-((5-chloro-2-((2-methoxy-4-(4-methylpiperazin-1-yl)phenyl)amino)pyrimidin-4-yl)methyl)-4-methylpiperidin-4-yl)cyclopropanesulfonamide (TDM-181184) was synthesized by a method similar to that of Example 9, as a yellow solid (8.8 mg, yield: 12.4%).

| TDM NO. | Structure | LCMS $[M+1]^+$ | $^1$H-NMR |
|---|---|---|---|
| TDM-181184 | | 564.2 | $^1$H NMR (400 MHz, DMSO-d6) δ 8.31 (s, 1H), 8.22 (s, 2H), 7.59 (d, J = 8.7 Hz, 1H), 6.66 (s, 1H), 6.61 (d, J = 2.6 Hz, 1H), 6.47 (dd, J = 8.7, 2.6 Hz, 1H), 3.77 (s, 3H), 3.49 (s, 2H), 3.19 (s, 2H), 3.12 (t, J = 5.0 Hz, 4H), 2.57 (dd, J = 14.4, 6.5 Hz, 3H), 2.46 (t, J = 4.9 Hz, 4H), 2.23 (s, 3H), 1.93 - 1.84 (m, 2H), 1.50 (t, J = 10.4 Hz, 2H), 1.33 (d, J = 2.7 Hz, 3H), 0.93 (d, J = 6.3 Hz, 4H). |

**Example 10: General Method for Synthesizing Compound 585 (TDM-181185)**

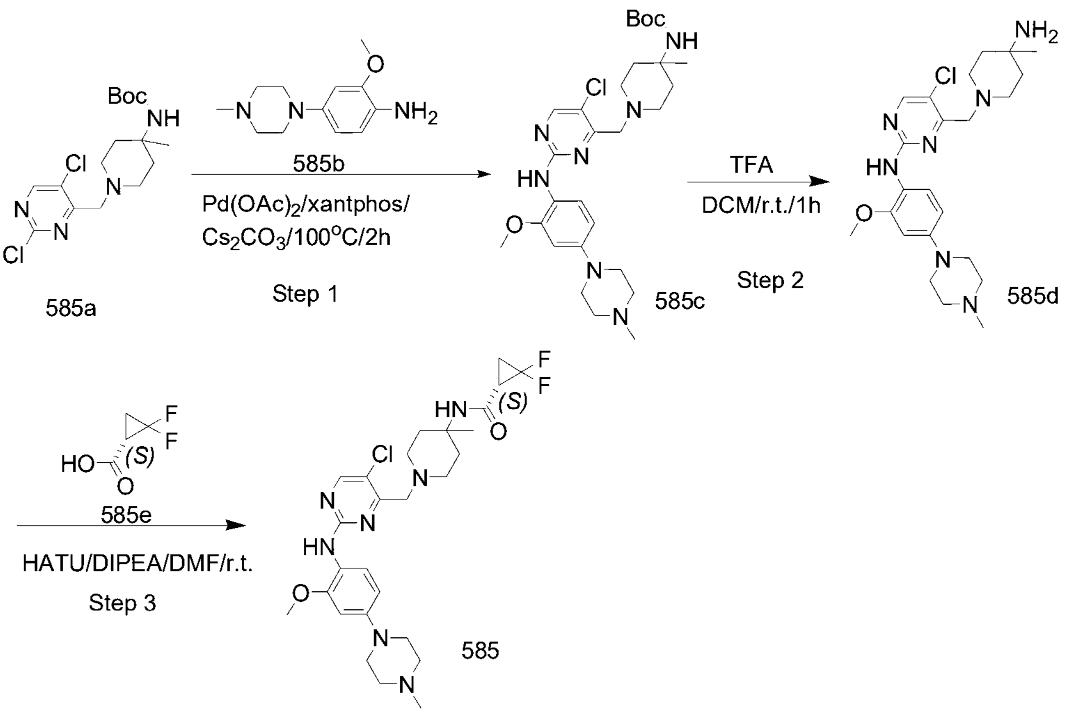


**Step 1: Compound 585c**

**Tert-butyl(1-((5-chloro-2-((2-methoxy-4-(4-methylpiperazin-1-yl)phenyl)amino)pyrimidin-4-yl)methyl)-4-methylpiperidin-4-yl)carbamate**

1,4-dioxane (10 mL) was added to a mixture of compound 585a (116 mg, 0.294 mmol), compound 585b (62 mg, 0.279 mmol), palladium acetate (13 mg, 0.0588 mmol), Xantphos (68 mg, 0.1176 mmol), and cesium carbonate (191 mg, 0.588 mmol) at room temperature. The mixture was heated to 100 °C and stirred for 2 hours. The mixture was concentrated under reduced pressure, and the residue was purified by silica gel chromatography (dichloromethane/10% methanol in dichloromethane = 0-80/20) to obtain the product (compound 585c, 145 mg, yield: 87.9%) as a white solid. LCMS $[M+1]^+$ = 560.

**Step 2: Compound 585d**

**4-((4-Amino-4-methylpiperidin-1-yl)methyl)-5-chloro-N-(2-methoxy-4-(4-methylpiperazin-1-yl)phenyl)pyrimidin-2-amine**

Trifluoroacetic acid (1.5 mL) was added to a solution of compound 585c (145 mg, 0.315 mmol) in dichloromethane (10 mL) at room temperature. The mixture was then stirred for 1 hour at 20°C. Water was added to the mixture and the mixture was neutralized with an aqueous solution of potassium carbonate and extracted with ethyl acetate, organic

layers were combined and dried over sodium sulfate, the filtrate was concentrated under reduced pressure, and the residue was used directly for the next reaction. LCMS $[M+1]^+$ = 460.

**Step 3: Compound 585**

**(S)-N-(1-((5-chloro-2-((2-methoxy-4-(4-methylpiperazin-1-yl)phenyl)amino)pyrimidin-4-yl)methyl)-4-methylpiperidin-4-yl)-2,2-difluorocyclopropane-1-carboxamide**

Compound 585e (21.9 mg, 0.179 mmol), 2-(7-azabenzotriazol-1-yl)-N,N,N',N'-tetramethyluronium hexafluorophosphate (68 mg, 0.179 mmol), and N,N-diisopropylethylamine (23 mg, 0.179 mmol) were added to a solution of compound 585d (55 mg, 0.120 mmol) in N,N-dimethylformamide (5 mL) at room temperature. The mixture was stirred for 1 hour. The mixture was concentrated under reduced pressure, and the residue was purified by silica gel chromatography (dichloromethane/10% methanol in dichloromethane = 0-100/0) and preparative HPLC (formic acid) to obtain the product (compound 585, TDM-181185, 10 mg, yield: 14.8%) as a yellow solid. LCMS $[M+1]^+$ = 564.3.

$^1H$ NMR (400 MHz, DMSO) δ 8.31 (s, 1H), 8.24 (s, 1H), 8.15 (s, 2H), 7.75 (s, 1H), 7.59 (d, J = 8.7 Hz, 1H), 6.62 (d, J = 2.4 Hz, 1H), 6.47 (dd, J = 8.8, 2.4 Hz, 1H), 3.77 (s, 3H), 3.49 (s, 2H), 3.18 - 3.11 (m, 4H), 2.67 (td, J = 6.5, 4.4 Hz, 1H), 2.62 (dd, J = 8.8, 5.8 Hz, 2H), 2.57 - 2.54 (m, 4H), 2.42 - 2.34 (m, 2H), 2.30 (s, 3H), 2.05 (s, 2H), 1.88 - 1.70 (m, 2H), 1.47 (dd, J = 11.2, 6.6 Hz, 2H), 1.25 (s, 3H).

**Example 11: General Method for Synthesizing Compound 586 (TDM-181186)**

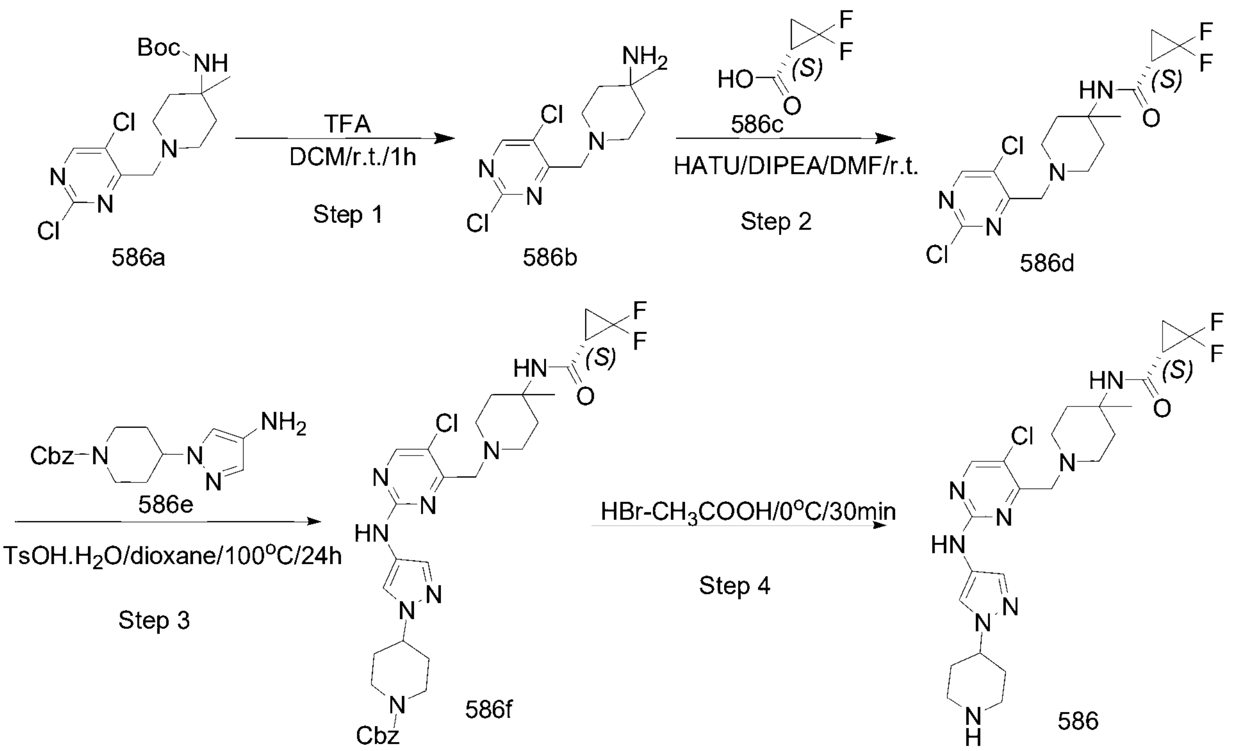


**Step 1: Compound 586b**

**1-((2,5-Dichloropyrimidin-4-yl)methyl)-4-methylpiperidin-4-amine**

Trifluoroacetic acid (2 mL) was added to a solution of compound 586a (116 mg, 0.309 mmol) in dichloromethane (10 mL) at room temperature. The mixture was stirred for 1 hour. Water was added, and the mixture was neutralized with aqueous sodium hydroxide solution and extracted with ethyl acetate. The organic layers were combined, dried over sodium sulfate, and the filtrate was concentrated under reduced pressure. The residue was purified by silica gel chromatography (dichloromethane/10% methanol ($NH_3$) in dichloromethane = 0-100/0) to obtain the product (compound 586b, 68 mg, yield: 80%) as a white solid. LCMS $[M+1]^+$ = 275 & 277.

**Step 2: Compound 586d**

**(S)-N-(1-((2,5-dichloropyrimidin-4-yl)methyl)-4-methylpiperidin-4-yl)-2,2-difluorocyclopropane-1-carboxamide**

Compound 586c (32 mg, 0.262 mmol), 2-(7-azabenzotriazol-1-yl)-N,N,N',N'-tetramethyluronium hexafluorophosphate (100 mg, 0.262 mmol), and N,N-diisopropylethylamine (34 mg, 0.262 mmol) were added to a solution of compound 586b (48 mg, 0.1475 mmol) in N,N-dimethylformamide (5 mL) at room temperature. The mixture was stirred for 1 hour. The mixture was added into water and extracted with ethyl acetate. The organic layers were combined, washed with brine, dried over sodium sulfate, and the filtrate was concentrated under reduced pressure. The residue was purified by silica gel chromatography (dichloromethane/10% methanol in dichloromethane = 0-15/85) to obtain the product (compound 586d, 56.8 mg, yield: 85.6%) as a white solid. LCMS $[M+1]^+$ = 379 & 381.

**Step 3: Compound 586f**

**Benzyl(S)-4-(4-((5-chloro-4-((4-(2,2-difluorocyclopropane-1-carboxamido)-4-methylpiperidin-1-yl)methyl)pyrimidin-2-yl)amino)-1H-pyrazol-1-yl)piperidine-1-carboxylate**

Compound 586e (41 mg, 0.136 mmol) and p-toluenesulfonic acid monohydrate (47 mg, 0.248 mmol) were added to a solution of compound 586d (47 mg, 0.124 mmol) in dioxane (5 mL) at room temperature. The mixture was heated to 100 °C and stirred for 24 hours. The mixture was added into water and extracted with ethyl acetate. The organic layers were combined, washed with brine, dried over sodium sulfate, and the filtrate was concentrated under reduced pressure. The residue was purified by silica gel chromatography (dichloromethane/10% methanol in dichloromethane = 0-50/50) to obtain the product (compound 586f, 21 mg, yield: 26.3%) as a yellow solid. LCMS $[M+1]^+$ = 643.

**Step 4: Compound 586**

**(S)-N-(1-((5-chloro-2-((1-(piperidin-4-yl)-1H-pyrazol-4-yl)amino)pyrimidin-4-yl)methyl)-4-methylpiperidin-4-yl)-2,2-difluorocyclopropane-1-carboxamide**

Compound 586f (21 mg, 0.033 mmol) was dissolved in HBr-AcOH (1.5 mL) at 0 °C, and the mixture was stirred at 0 °C for 30 minutes. The mixture was concentrated under reduced pressure at 45 °C, and the residue was purified by preparative HPLC (formic acid) to obtain the product (compound 586, TDM-181186, 6.7 mg, yield: 40.4%) as a gray solid. LCMS $[M+1]^+$ = 509.

$^1H$ NMR (400 MHz, DMSO) δ 9.74 (s, 1H), 8.39 (s, 1H), 8.23 (s, 2H), 7.96 (s, 1H), 7.79 (s, 1H), 7.59 (s, 1H), 4.39 (d, J = 10.4 Hz, 1H), 4.10 - 3.99 (m, 1H), 3.54 (s, 2H), 3.34 (d, J = 12.3 Hz, 2H), 2.98 (t, J = 11.0 Hz, 2H), 2.73 - 2.66 (m, 1H), 2.65 - 2.55 (m, 2H), 2.39 (s, 2H), 2.08 (dd, J = 27.2, 12.6 Hz, 6H), 1.88 - 1.70 (m, 2H), 1.48 (d, J = 8.9 Hz, 2H), 1.26 (s, 3H).

**Test Example: Enzyme Activity Inhibition Test of NUAK1/NUAK2 Kinase Inhibitors**

The inhibitory activity of the pyrimidinamine small molecules involved in the present application against NUAK1 and NUAK2 kinases was determined using a kinase activity assay based on the P81 filter paper binding hot-spot technique (Nat Biotechnol. 2011, 29:1039). A brief description is as follows:

The assay buffer system contained 20 mM Hepes (pH 7.5), 10 mM $MgCl_2$, 1 mM EGTA, 0.01% Brij35 (L23 polyoxyethylene lauryl ether), 0.02 mg/mL BSA (bovine serum albumin), 0.1 mM $Na_3VO_4$, 2 mM DTT, and 1% DMSO.

Test Steps:

1. A 20 μM substrate solution was prepared with freshly prepared test buffer and CHKtide peptide fragments (Sanchez Y. Science. 1997, 277: 1497-1501) were used as a substrate.
2. Kinases were added and mixed gently (a final concentration of NUAK1 was 10 nM; and a final concentration of NUAK2 was 50 nM).
3. Test compounds dissolved in 100% DMSO were each added to the above kinase reaction mixture solution via Acoustic Droplet Ejection (Echo550; nanoliter) and the mixture was incubated for 5 minutes at room temperature.
4. $^{33}P$-labeled ATP (a ratio of unlabeled ATP to labeled ATP was 25:1 or 2.5:1) was added.
5. The mixture was incubated for 2 hours at room temperature.
6. Kinase activity was tested using the P81 filter paper combined with hotspot technology.

The $IC_{50}$ values of the test compounds against NUAK1/NUAK2 were calculated according to the above test. For specific results, see Table 1.

The $IC_{50}$ values were calculated by using a formula derived from sigmoidal dose-response curve (variable slope):

$$Y = Bottom + (Top-Bottom)/(1+10^{\wedge}((LogEC50-X)*Slope,$$

where X is the logarithm of compound concentration, Y is the response (inhibition rate of kinase activity), and Y rises from bottom to top along the sigmoidal curve as the concentration increases.

Table 1: $IC_{50}$ (nM) of Test Compounds of the Present Application against NUAK1/NUAK2

| Test Compound | NUAK1 | NUAK2 |
| --- | --- | --- |
| TDM-181137 | 71 | 29 |
| TDM-181163 | 30 | 15 |
| TDM-181164 | 6 | 15 |
| TDM-181165 | 39 | 72 |
| TDM-181172 | 3 | 4 |
| TDM-181173 | 1 | 3 |
| TDM-181174 | 0.46 | 1.44 |
| TDM-181175 | 20 | 13 |
| TDM-181178 | 1.24 | 2.76 |
| TDM-181182 | 679 | Untested |
| TDM-181183 | 69 | Untested |
| TDM-181186 | 959 | Untested |

As can be seen from the results in Table 1, the pyrimidinamine-based compounds of the present application exhibit excellent inhibitory activity against both NUAK1 and NUAK2, and are dual-target small-molecule kinase inhibitors. The $IC_{50}$ values of the compounds against NUAK1 can be less than 1 nM, and the $IC_{50}$ values against NUAK2 can reach several nM to several tens of nM. Therefore, the above experiments demonstrate that the pyrimidinamine-based compounds of the present application can be used as NUAK1/NUAK2 inhibitors.

The preferred embodiments of the present disclosure have been described in detail above. However, the present disclosure is not limited to the specific details in the above embodiments. Within the scope of the technical concept of the present disclosure, various simple modifications can be made to the technical solutions of the present disclosure, and all such simple modifications fall within the scope of protection of the present disclosure.

In addition, it should be noted that the various specific technical features described in the above specific embodiments may be combined in any suitable manner without contradiction. To avoid unnecessary repetition, the present disclosure does not separately describe every possible combination.

Moreover, any combination of the various embodiments of the present disclosure may be made, provided that such combination does not depart from the spirit of the present disclosure, and such combinations shall also be regarded as the content disclosed in the present disclosure.

## Claims

1. A pyrimidinamine-based compound, wherein the pyrimidinamine-based compound is a compound represented by Formula I, or a stereoisomer, a tautomer, an isotopic derivative, a hydrate, a solvate, a prodrug, or a pharmaceutically acceptable salt thereof,

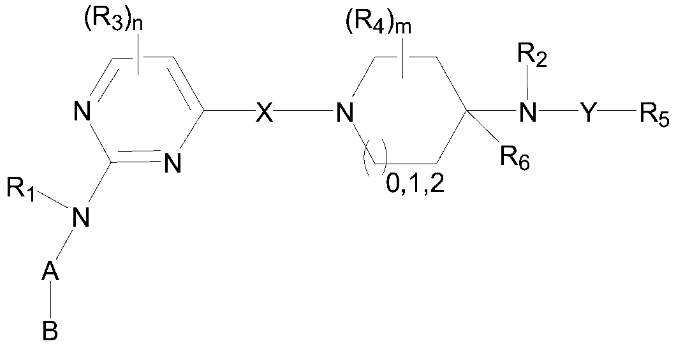

Formula I

wherein X is a direct bond, -O-, -S-, $-NR_7-$ or $-CR_8R_9-$, and Y is -CO- or $-S(O)_2-$;

A is an optionally substituted 6- to 10-membered aryl group or 5- to 10-membered heteroaryl group, B is an optionally substituted 3- to 10-membered cycloalkyl group or 3- to 10-membered heterocycloalkyl group, and the substituent on A or B is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;

$R_1$, $R_2$, $R_6$, $R_7$, $R_8$, and $R_9$ are each independently H or an optionally substituted C1-C8 alkyl group;

$R_3$ and $R_4$ are each independently halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, or an optionally substituted C1-C8 alkylamino group;

$R_5$ is an optionally substituted 3- to 10-membered cycloalkyl group or an optionally substituted 3- to 10-membered heterocycloalkyl group, wherein the substituent on the cycloalkyl group or heterocycloalkyl group is one or more selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;

the substituent on the C1-C8 alkyl group is one or more selected from the group consisting of halogen, an amino group, a hydroxyl group, a nitro group, a cyano group, and a sulfhydryl group; and

n is 0, 1, or 2, and m is 0, 1, 2, 3, 4, or 5.

**2.** The compound according to claim 1, wherein $R_5$ is an optionally substituted 3- to 10-membered cycloalkyl group.

**3.** The compound according to claim 2, wherein $R_5$ is an optionally substituted 3- to 6-membered cycloalkyl group.

**4.** The compound according to claim 3, wherein $R_5$ is a 3- to 6-membered cycloalkyl group substituted with halogen.

**5.** The compound according to claim 4, wherein $R_5$ is a 3- to 6-membered cycloalkyl group substituted with fluorine.

**6.** The compound according to claim 3, wherein $R_5$ is

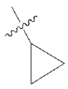

or

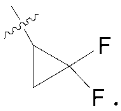

.

**7.** The compound according to claim 6, wherein $R_5$ is

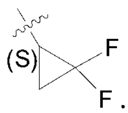

.

**8.** The compound according to any one of claims 1 to 7, wherein $R_6$ is a methyl group.

**9.** The compound according to any one of claims 1 to 8, wherein $R_2$ is H.

**10.** The compound according to any one of claims 1 to 9, wherein B is an optionally substituted 3- to 6-membered heterocycloalkyl group.

**11.** The compound according to claim 10, wherein B is an optionally substituted 5- to 6-membered heterocycloalkyl group.

**12.** The compound according to claim 11, wherein B is an optionally substituted piperidinyl group or an optionally substituted piperazinyl group.

**13.** The compound according to claim 12, wherein the compound represented by Formula I is represented by Formula I-1 or Formula I-2:

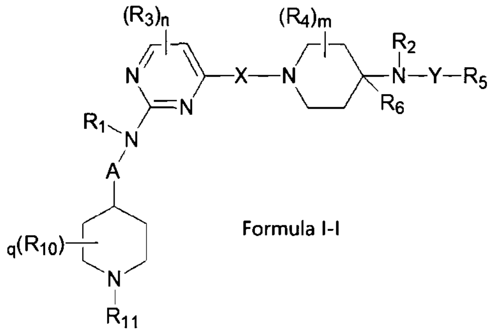

Formula I-l

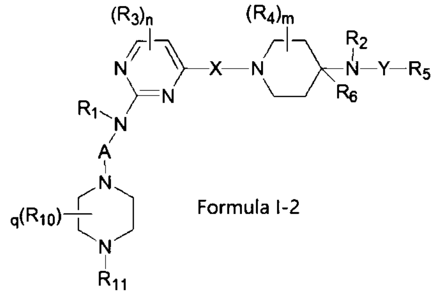

Formula I-2

,

$R_{10}$ is selected from the group consisting of halogen, an amino group, a hydroxyl group, a nitro group, a cyano group, a sulfhydryl group, an optionally substituted C1-C8 alkyl group, an optionally substituted C1-C8 alkoxy group, an optionally substituted C1-C8 alkylthio group, and an optionally substituted C1-C8 alkylamino group;
$R_{11}$ is H or an optionally substituted C1-C8 alkyl group;
the substituent on the C1-C8 alkyl group is one or more selected from the group consisting of halogen, an amino group, a hydroxyl group, a nitro group, a cyano group, and a sulfhydryl group; and
q is 0, 1, 2, 3, or 4.

**14.** The compound according to claim 13, wherein $R_{11}$ is H or a methyl group.

**15.** The compound according to any one of claims 1 to 14, wherein A is an optionally substituted phenyl group or an optionally substituted 5-membered heteroaryl group.

**16.** The compound according to claim 15, wherein A is one of the following optionally substituted moieties:

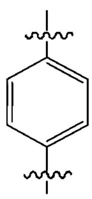
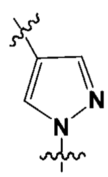
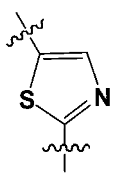

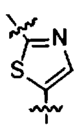
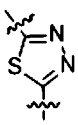
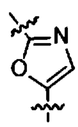
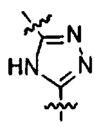
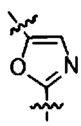
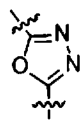
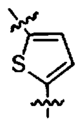

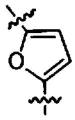 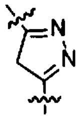 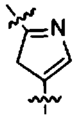 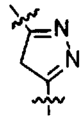 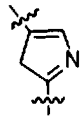 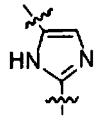 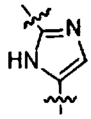

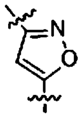 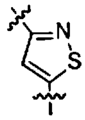 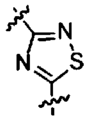 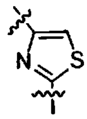 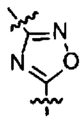 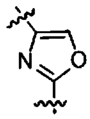

**17.** The compound according to any one of claims 1 to 16, wherein $R_3$ is chloro, preferably at the para-position to the amino group.

**18.** The compound according to any one of claims 1 to 17, wherein the substituent on ring A is a methoxy group, preferably at the ortho-position to the amino group.

**19.** The compound according to any one of claims 1 to 18, wherein the compound represented by Formula I is one of the following compounds:

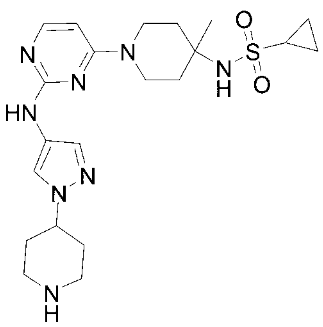
TDM-181137

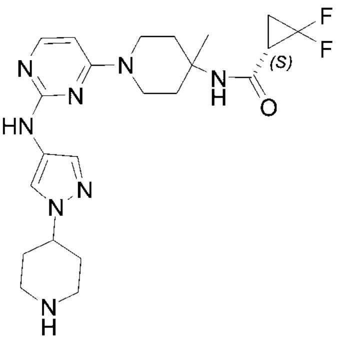
TDM-181163

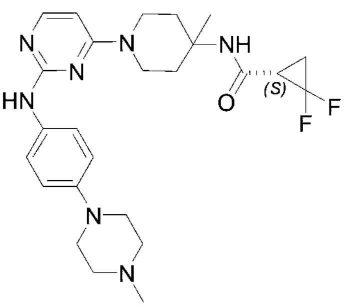
TDM-181164

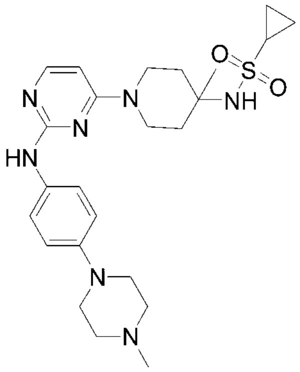
TDM-181165

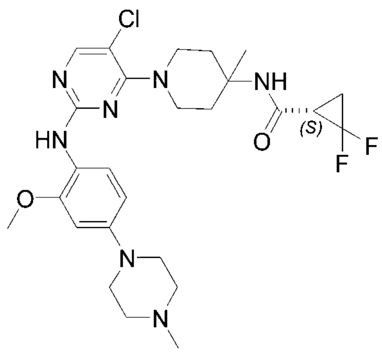
TDM-181172

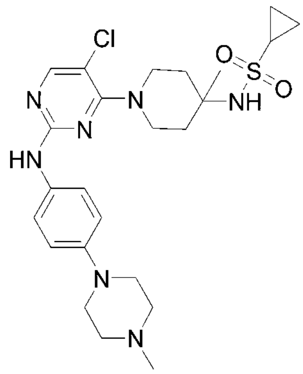
TDM-181173

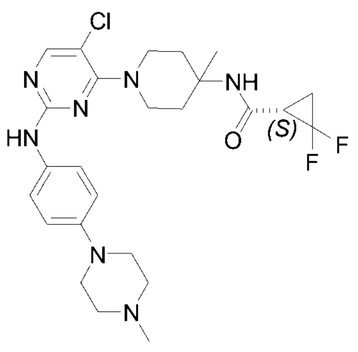

TDM-181174

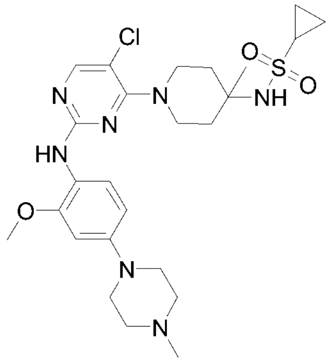

TDM-181175

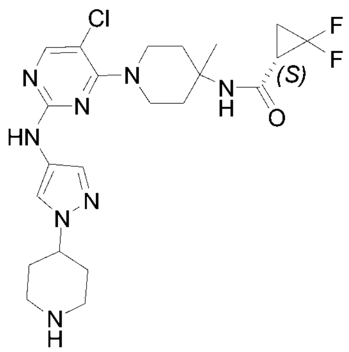

TDM-181178

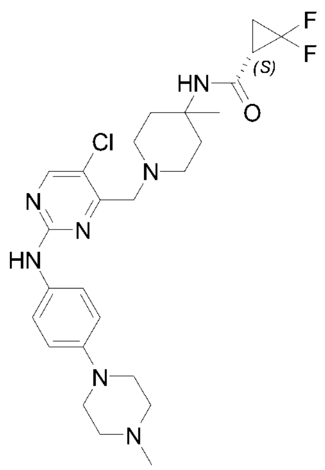

TDM-181182

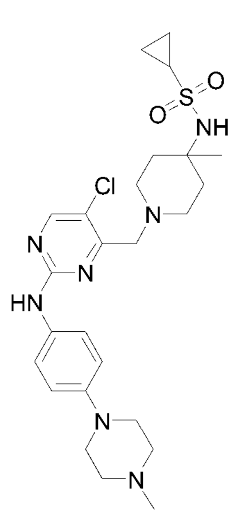

TDM-181183

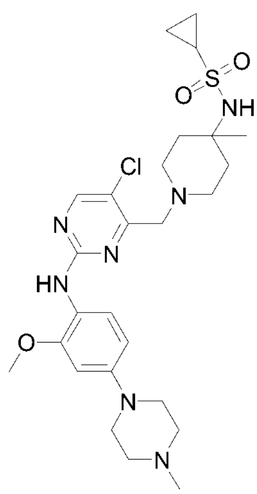

TDM-181184

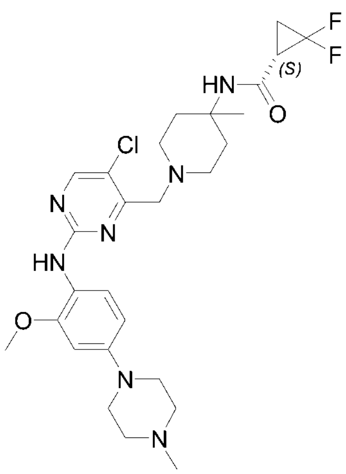

TDM-181185

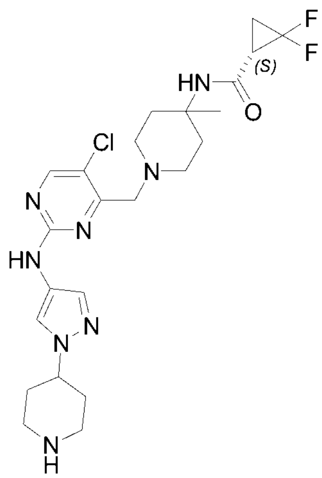

TDM-181186

.

**20.** The compound according to any one of claims 1 to 19, wherein the pharmaceutically acceptable salt is a formate salt.

**21.** The compound according to any one of claims 1 to 20, wherein the isotopic derivative is a deuterated substance.

**22.** A method for preparing the compound according to any one of claims 1 to 21, comprising:

preparing the compound of Formula I from a compound of Formula II and a compound of Formula III; or

preparing a compound of Formula V from a compound of Formula IV and a compound of Formula II, and then preparing the compound of Formula I from the compound of Formula V;

optionally, the preparation process comprises necessary protection and deprotection steps;

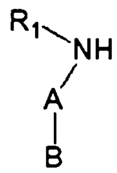

Formula II

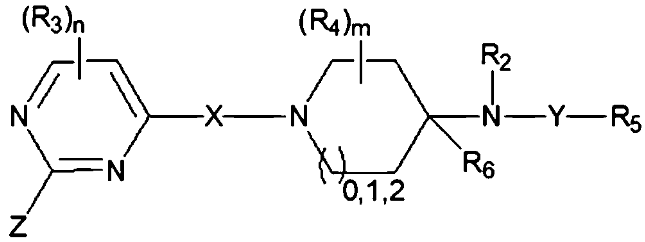

Formula III

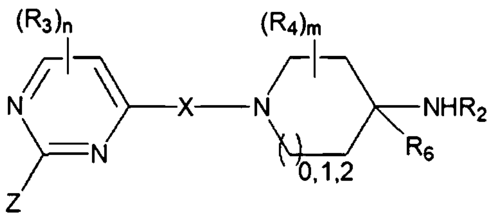

Formula IV

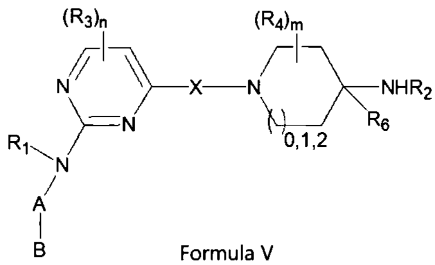

Formula V

wherein Z is a leaving group, preferably halogen, more preferably Cl, and other groups are as defined in claims 1 to 21.

**23.** A pharmaceutical composition comprising the compound according to any one of claims 1 to 21 as an active ingredient.

**24.** The pharmaceutical composition according to claim 23, comprising a pharmaceutically acceptable carrier or excipient.

**25.** Use of the compound according to any one of claims 1 to 21 in preparation of a NUAK1 or NUAK2 inhibitor.

**26.** Use of the compound according to any one of claims 1 to 21 in preparation of a medicament, wherein the compound is used for the prevention or treatment of the following diseases by inhibiting NUAK1 or NUAK2: neuropsychiatric diseases, metabolic diseases, tumors, visceral fibrotic diseases, or skin fibrotic diseases.

**27.** The use according to claim 26, wherein the disease is Parkinson's disease, Alzheimer's disease, diabetes, hyperlipidemia, obesity, liver cancer, leukemia, lymphoma, tumor metastasis, liver cirrhosis, renal fibrosis, pulmonary fibrosis, post-myocarditis sequelae, scleroderma, keloids, or hypertrophic scar, or the compound is used solely for alleviating a traumatic or postoperative scar.

## INTERNATIONAL SEARCH REPORT

International application No.
**PCT/CN2024/103753**

**A. CLASSIFICATION OF SUBJECT MATTER**

C07D 401/14(2006.01)i; C07D 401/04(2006.01)i; A61K 31/506(2006.01)i; A61P 25/00(2006.01)i; A61P 25/16(2006.01)i; A61P 25/28(2006.01)i; A61P 3/00(2006.01)i; A61P 3/10(2006.01)i; A61P 3/06(2006.01)i; A61P 3/04(2006.01)i; A61P 35/00(2006.01)i; A61P 35/02(2006.01)i; A61P 13/12(2006.01)i; A61P 11/00(2006.01)i; A61P 1/16(2006.01)i; A61P 9/00(2006.01)i; A61P 17/00(2006.01)i

According to International Patent Classification (IPC) or to both national classification and IPC

**B. FIELDS SEARCHED**

Minimum documentation searched (classification system followed by classification symbols)

IPC:C07D A61K A61P

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN: 特科罗生物科技, 方文奎, 李冠群, 蔡雨婷, 王增全, 嘧啶, 嘧啶胺, NUAK, 蛋白激酶, 肿瘤, 癌症, Pyrimidin+, Pyrimidinamine, Protein kinas, cancer, tumor, 结构式

**C. DOCUMENTS CONSIDERED TO BE RELEVANT**

| Category* | Citation of document, with indication, where appropriate, of the relevant passages | Relevant to claim No. |
|---|---|---|
| PX | CN 116903592 A (TECHNODERMA MEDICINES INC.) 20 October 2023 (2023-10-20) claims 1-27 | 1-27 |
| PA | CN 117088851 A (TECHNODERMA MEDICINES INC.) 21 November 2023 (2023-11-21) claims 1-28 | 1-27 |
| PA | CN 116947764 A (TECHNODERMA MEDICINES INC.) 27 October 2023 (2023-10-27) claims 1-22 | 1-27 |
| A | WO 2023279105 A1 (AERIE PHARMACEUTICALS, INC.) 05 January 2023 (2023-01-05) claims 1-27, and description, page 130 | 1-27 |
| A | WO 2009017838 A2 (EXELIXIS, INC. et al.) 05 February 2009 (2009-02-05) claims 1-19, and description, paragraphs [0001]-[0004] | 1-27 |
| A | CN 114401722 A (BRIDGENE BIOSCIENCES, INC.) 26 April 2022 (2022-04-26) claims 1-23, and description, paragraph [0252] | 1-27 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 September 2024** | **01 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/103753**

**C. DOCUMENTS CONSIDERED TO BE RELEVANT**

| Category* | Citation of document, with indication, where appropriate, of the relevant passages | Relevant to claim No. |
|---|---|---|
| A | CN 113226462 A (BOEHRINGER INGELHEIM INTERNATIONAL GMBH) 06 August 2021 (2021-08-06)<br>entire document | 1-27 |
| A | CN 107074867 A (PFIZER INC.) 18 August 2017 (2017-08-18)<br>entire document | 1-27 |
| A | WO 2022184130 A1 (HITGEN INC.) 09 September 2022 (2022-09-09)<br>entire document | 1-27 |
| A | CN 115838370 A (SHANDONG NEW TIME PHARMACEUTICAL CO., LTD.) 24 March 2023 (2023-03-24)<br>entire document | 1-27 |
| A | CN 1989138 A (CYCLACEL LIMITED) 27 June 2007 (2007-06-27)<br>entire document | 1-27 |

Form PCT/ISA/210 (second sheet) (July 2022)

**INTERNATIONAL SEARCH REPORT**
**Information on patent family members**

International application No.
**PCT/CN2024/103753**

| Patent document cited in search report | | | Publication date (day/month/year) | Patent family member(s) | | | Publication date (day/month/year) |
|---|---|---|---|---|---|---|---|
| CN | 116903592 | A | 20 October 2023 | None | | | |
| CN | 117088851 | A | 21 November 2023 | None | | | |
| CN | 116947764 | A | 27 October 2023 | None | | | |
| WO | 2023279105 | A1 | 05 January 2023 | CA | 3225872 | A1 | 05 January 2023 |
| | | | | AU | 2022304597 | A1 | 04 January 2024 |
| | | | | CN | 117642395 | A | 01 March 2024 |
| | | | | KR | 20240031347 | A | 07 March 2024 |
| | | | | IN | 202417005745 | A | 15 March 2024 |
| | | | | BR | 112023027493 | A2 | 26 March 2024 |
| | | | | EP | 4363058 | A1 | 08 May 2024 |
| | | | | MX | 2024000273 | A1 | 03 January 2024 |
| | | | | RU | 2023132820 | A | 07 March 2024 |
| | | | | JP | 2024525368 | A | 12 July 2024 |
| WO | 2009017838 | A2 | 05 February 2009 | WO | 2009017838 | A3 | 02 July 2009 |
| CN | 114401722 | A | 26 April 2022 | EP | 3999064 | A1 | 25 May 2022 |
| | | | | EP | 3999064 | A4 | 28 June 2023 |
| | | | | CA | 3147741 | A1 | 28 January 2021 |
| | | | | JP | 2022541274 | A | 22 September 2022 |
| | | | | US | 2022288069 | A1 | 15 September 2022 |
| | | | | AU | 2020316011 | A1 | 03 March 2022 |
| | | | | WO | 2021016102 | A1 | 28 January 2021 |
| | | | | HK | 40075155 | A0 | 13 January 2023 |
| CN | 113226462 | A | 06 August 2021 | MX | 2021006489 | A | 06 July 2021 |
| | | | | EP | 3890828 | A1 | 13 October 2021 |
| | | | | US | 2022048889 | A1 | 17 February 2022 |
| | | | | KR | 20210099093 | A | 11 August 2021 |
| | | | | BR | 112021010823 | A2 | 24 August 2021 |
| | | | | CA | 3120037 | A1 | 11 June 2020 |
| | | | | WO | 2020114947 | A1 | 11 June 2020 |
| | | | | JP | 2022510351 | A | 26 January 2022 |
| | | | | JP | 7195436 | B2 | 23 December 2022 |
| | | | | AU | 2019392524 | A1 | 15 July 2021 |
| CN | 107074867 | A | 18 August 2017 | TW | 201609706 | A | 16 March 2016 |
| | | | | US | 2016052930 | A1 | 25 February 2016 |
| | | | | US | 9663526 | B2 | 30 May 2017 |
| | | | | ES | 2655971 | T3 | 22 February 2018 |
| | | | | ES | 2655971 | T9 | 14 September 2018 |
| | | | | ZA | 201701005 | B | 31 July 2019 |
| | | | | AU | 2015304883 | A1 | 02 March 2017 |
| | | | | AU | 2015304883 | B2 | 10 May 2018 |
| | | | | CA | 2900855 | A1 | 21 February 2016 |
| | | | | CA | 2900855 | C | 15 March 2022 |
| | | | | PH | 12017500276 | A1 | 03 July 2017 |
| | | | | AR | 101599 | A1 | 28 December 2016 |
| | | | | WO | 2016027195 | A1 | 25 February 2016 |
| | | | | KR | 101877189 | B1 | 10 July 2018 |
| | | | | MY | 192109 | A | 27 July 2022 |
| | | | | BR | 112017003054 | A2 | 21 November 2017 |
| | | | | BR | 112017003054 | B1 | 11 April 2023 |

Form PCT/ISA/210 (patent family annex) (July 2022)

**INTERNATIONAL SEARCH REPORT**
**Information on patent family members**

International application No.
**PCT/CN2024/103753**

| Patent document cited in search report | Publication date (day/month/year) | Patent family member(s) | Publication date (day/month/year) |
|---|---|---|---|
| | | US 10463675 B2 | 05 November 2019 |
| | | JP 2017524022 A | 24 August 2017 |
| | | JP 6218260 B2 | 25 October 2017 |
| | | IL 250709 B | 30 May 2019 |
| | | MX 2017002241 A | 03 May 2017 |
| | | EA 032609 B1 | 28 June 2019 |
| | | EP 3183247 A1 | 28 June 2017 |
| | | EP 3183247 B1 | 29 November 2017 |
| | | EP 3183247 B9 | 25 July 2018 |
| | | SG 11201700957 A1 | 30 March 2017 |
| | | TW 583681 B1 | 21 May 2017 |
| | | VN 52443 A | 25 May 2017 |
| | | MD 20170016 A2 | 31 August 2017 |
| | | ID 201713399 A | 08 December 2017 |
| | | HK 1241857 A0 | 15 June 2018 |
| | | GC 8944 A | 31 October 2018 |
| | | GE 201907003 B | 25 July 2019 |
| | | CN 107074867 B | 22 October 2019 |
| | | SG 11201700957 B | 09 April 2020 |
| | | NZ 729005 A2 | 26 June 2020 |
| | | HK 1241857 A1 | 11 December 2020 |
| | | VN 10028550 B | 25 June 2021 |
| | | IN 436732 B | 07 July 2023 |
| WO 2022184130 A1 | 09 September 2022 | CN 115141195 A | 04 October 2022 |
| | | CN 115141195 B | 06 February 2024 |
| CN 115838370 A | 24 March 2023 | WO 2023041071 A1 | 23 March 2023 |
| | | CN 116731020 A | 12 September 2023 |
| | | CN 118103371 A | 28 May 2024 |
| | | CN 116726022 A | 12 September 2023 |
| | | EP 4403556 A1 | 24 July 2024 |
| CN 1989138 A | 27 June 2007 | BRPI 0511616 A | 02 January 2008 |
| | | EP 1756098 A2 | 28 February 2007 |
| | | WO 2005116025 A2 | 08 December 2005 |
| | | WO 2005116025 A3 | 23 February 2006 |
| | | US 2009137572 A1 | 28 May 2009 |
| | | JP 2008500320 A | 10 January 2008 |
| | | JP 5026259 B2 | 12 September 2012 |
| | | AU 2005247682 A1 | 23 November 2006 |
| | | MX 2006013632 A1 | 01 April 2007 |
| | | IN 200606898 P1 | 31 August 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310860161


### Non-patent literature cited in the description


- **HARDIE DG**. *Trends in Cell Biology*, 2016, vol. 26, 190
- **STEINBERG GR**. *Nature Reviews Drug Discovery*, 2019, vol. 18, 527
- **BRIGHT NJ**. *Acta Physiologica.*, 2009, vol. 196, 15
- **SUN X**. *J of Molecular Endocrinology.*, 2013, vol. 51, R15
- **BROOKS D**. *Data in Brief*, 2022, vol. 43, 108482
- **BONNARD C**. *J Exp Med.*, 2020, vol. 217, e20191561
- **BENNISON SA**. *Cellular Signaling.*, 2022, vol. 100, 110472
- **BLAZEJEWSKI SM**. *Scientific Reports.*, 2011, vol. 11, 8156
- **HOU X**. *Oncogen*, vol. 201 (30), 2933
- **CHEN Y**. *Cell Death and Disease*, 2020, vol. 11, 712
- **MOLINA E**. *Cells*, vol. 10, 2760
- **HUMBERT N**. *The EMBO Journal*, 2010, vol. 29, 376
- **GILL MK**. *Nature Communications.*, 2018
- **VIS RAJ**. *Cancers*, 2021, vol. 13, 3377
- **BANERJEE S**. *The Biochemical Journal*, 2014, vol. 457, 215
- IUPAC 1974 Recommendations for Section E, Fundamental Stereochemistry. *Pure Appl. Chem.*, 1976, vol. 45, 13-10
- *Nat Biotechnol.*, 2011, vol. 29, 1039
- **SANCHEZ Y**. *Science*, 1997, vol. 277, 1497-1501